# EUROPEAN PATENT APPLICATION

(11) **EP 3 744 828 A1**
(43) Date of publication of application: **02.12.2020**
(21) Application number: 19177251.6
(22) Date of filing: 29.05.2019
(51) Int. Cl.: C12N 1/20, C12N 9/10, A61K 39/02

(54) **NEW IMMUNOGENIC COMPOSITIONS**

(71) Applicant: ETH Zurich, 8092 Zurich (CH)
(72) Inventor: WETTER, Emma, 8105 Watt (CH); DIARD, Médéric, 4051 Basel (CH); HARDT, Wolf-Dietrich, 8103 Unterengstringen (CH)

(57) **Abstract**

The present invention relates to an immunogenic composition for Proteobacteria protection and reduced transmission. We have identified Proteobacteria strain combinations that generate an immune response capable of robustly driving bacterial enteropathogens into an evolutionary dead end and reducing the transmission of the bacterium. These inactivated immunogenic compositions and typically oral vaccines are easy to apply, cheap to produce, and can be stored long-term without cold-chain requirements making them ideal for application in livestock, or in resource-poor areas. They are believed to be the only immunogenic compositions and vaccine formulations capable of breaking the chain of transmission for these types of pathogen.

## Description

The present invention relates to new immunogenic compositions and uses in the treatment and prevention of diseases caused by Proteobacteria.

### RELATED ART

Bacterial enteropathogens of the *Enterobacteriaceae* family account for two of the four key global causes of diarrheal diseases (WHO) and can cause life-threatening invasive disease or severe post-infectious sequela in susceptible individuals (Kirk, M. D. et al. World Health Organization Estimates of the Global and Regional Disease Burden of 22 Foodborne Bacterial, Protozoal, and Viral Diseases, 2010: A Data Synthesis. PLOS Med. 12, e1001921, 2015). As well as causing infections, these strains are very often asymptomatically carried in the intestinal microbiota, as well as gut-draining lymphoid tissues, of livestock, in Europe, which is a major concern in pigs and chickens (Niemann, J.-K. et al. Simultaneous occurrence of Salmonella enterica, Campylobacter spp. and Yersinia enterocolitica was observed along the pork production chain from farm to meat processing in five conventional fattening pig herds in Lower Saxony. Berl. Munch. Tierarztl. Wochenschr. 129, 296-303). These infection reservoirs fuel seasonal re-emergence of infections and continuous cycles of infection in young animals. Combined with the on-going requirement for antibiotics in disease management in livestock rearing (WHO), this represents a ticking time bomb with respect to the development of multidrug resistant zoonostic pathogens. There is therefore a pressing need for effective vaccines that block the infectious cycle.

A major hurdle to developing such vaccines has been our limited understanding of how protective immunity to bacterial pathogens is actually achieved in the intestine. It has long been known that secretory antibodies, i.e. sIgA, are the major immune component present in the gut lumen of vaccinated animals, and protection is typically mediated by their binding to the O-antigen of lipopolysaccharide (Endt, K. et al. The microbiota mediates pathogen clearance from the gut lumen after non-typhoidal salmonella diarrhea. PLoS Pathog. 6, e1001097 (2010)). However, this protection was observed often to be incomplete and to vary in efficiency depending on the level of exposure. sIgA-O-antigen interactions function by crosslinking cells as the bacteria grow and divide (Moor, K. et al. High-avidity IgA protects the intestine by enchaining growing bacteria. Nature 544, 498-502 (2017). This generates large clumps of bacteria that cannot approach the gut wall, preventing the delivery of virulence factors. However, being enchained in a clump also generates a selective pressure on the luminal enteropathogen population. This drives rapid evolution of vaccine-escape variants that no longer bind to vaccine-induced antibodies, accounting for the unsatisfactory percentage protection observed. These pathogen variants typically carry a chemical modification of the O-antigen (Broadbent, S. E., Davies, M. R. & van der Woude, M. W. Phase variation controls expression of Salmonella lipopolysaccharide modification genes by a DNA methylation-dependent mechanism. Mol. Microbiol. 77, 337-53 (2010) and Hauser, E., Junker, E., Helmuth, R. & Malorny, B. Different mutations in the oafA gene lead to loss of O5-antigen expression in Salmonella enterica serovar Typhimurium. J. Appl. Microbiol. 110, 248-53 (2011)).

The non-Typhoidal *Salmonella* (NTS) serotypes are a primary cause of foodborne illnesses worldwide. In the U.S. NTS are a leading cause of hospitalization and death due to foodborne illnesses, with *Salmonella enterica serovar Typhimurium* (S.Tm) being the most frequent cause. 95% of the total cases of NTS are caused by contaminated food. Unfortunately, absolute protection from infection by enhanced agricultural surveillance is not feasible. The rapid evolution of vaccine-escape variants of Proteobacteria is a major hurdle in vaccine development and explains why there are no successfully licensed vaccines for either human or large-animal use against non-Typhoidal *Salmonella* (NTS) and only very limited options available for pathogenic *E. coli.*

### SUMMARY OF THE INVENTION

The present invention relates in particular to an immunogenic composition for Proteobacteria protection and reduced transmission. We have identified Proteobacteria strain combinations that generate an immune response capable of robustly driving bacterial enteropathogens into an evolutionary dead end and reducing the transmission of the bacterium. These inactivated typically oral immunogenic compositions and vaccines, respectively, are easy to apply, cheap to produce, and can be stored long-term without cold-chain requirements making them ideal for application in livestock, or in resource-poor areas. According to our knowledge, these are the only immunogenic compositions and vaccine formulations, respectively, capable of breaking the chain of transmission for these types of pathogen.

Thus, in a first aspect, the present invention provides for an immunogenic composition comprising at least two or more inactivated serovar of a Proteobacteria strain, wherein each of said two or more inactivated serovar comprises a genetic modification of the O-antigen, wherein independently each of said genetic modification comprises a glucosylation and/or O-acetylation of the O-antigen.

In a further aspect, the present invention provides for the inventive immunogenic composition for use as a prophylactic treatment against a disease caused by Proteobacteria in a subject, wherein preferably the subject following treatment contains only a non-transmissible form of said Proteobacteria.

In a further aspect, the present invention provides for the inventive immunogenic composition for use in a method of inducing an immune response against a disease caused by Proteobacteria in a subject, wherein said method comprises administrating said immunogenic composition to said subject in need thereof, and wherein preferably administration is by intranasal, intramuscular, subcutaneous, transdermal or sublingual administration.

In a further aspect, the present invention provides for the inventive immunogenic composition for use in a method of preventing disease caused by Proteobacteria selected from the group consisting of bacterial gastroenteritis, bacterial enterocolitis, urinary tract infection, mastitis, bacterial pneumonia and bacterial sepsis.

In another aspect, the present invention provides for the method of generating the inventive immunogenic composition, wherein said method comprises the following steps:
i. Administer an inactivated wild type Proteobacterial strain to a subject,
ii. Challenge the subject with the wildtype Proteobacterial strain,
iii. Isolate clones of said Proteobacterial strain from the subject between 2 hours to 7 days post infection,
iv. Identify isolated clones with reduced binding affinity to the wild type Proteobacteria anti-O-antigen antibody,
v. Generate recombinant clones identical to said isolated clones with reduced binding affinity,
vi. Inactivate and combine the recombinant clones with the inactivated wild type Proteobacterial strain,
vii. Repeat steps i-viii, administering the inactivated combined recombinant clones with the inactivated wild-type Proteobacterial strain,
viii. Combine all identified inactivated clones with the inactivated wild type Proteobacteria to produce the immunogenic composition.

Further aspects and embodiments of the present invention will become apparent as this description continues.

### DESCRIPTION OF FIGURES

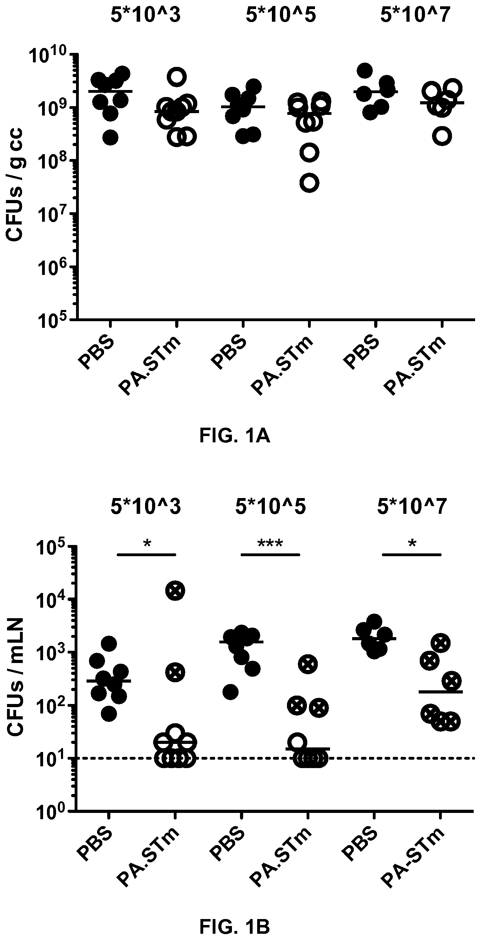

- FIG. 1A:: Graph shows the fecal *Salmonella* Colony Forming Units per gram cecal content (CFU/gcc) of mice that were either vaccinated with 1e10 particles of peracetic acid inactivated wild-type *S. Typhimurium* strain SL1344 (PA.STm) or vehicle-only control (PBS). The three test groups of mice were orally challenged with live wild-type *S.* Typhimurium strain SL1344 with an inoculum size of 5^{∗}10³, 5^{∗}10⁵, and 5^{∗}10⁷. The graph demonstrates protection failure of a wild-type *Salmonella* Typhimurium vaccine.
- FIG. 1B:: Graph shows the lymph node *Salmonella* Colony Forming Units per mesenteric lymph node (CFU/mLN) of mice that were either vaccinated with 1e10 particles of peracetic acid inactivated wild-type *S. Typhimurium* strain SL1344 (PA.STm) or vehicle-only control (PBS). The three test groups of mice were orally challenged with live wild-type S. Typhimurium strain SL1344 with an inoculum size of 5^{∗}10³, 5^{∗}10⁵, and 5^{∗}10⁷. The graph demonstrates protection failure of a wild-type *Salmonella* Typhimurium vaccine.

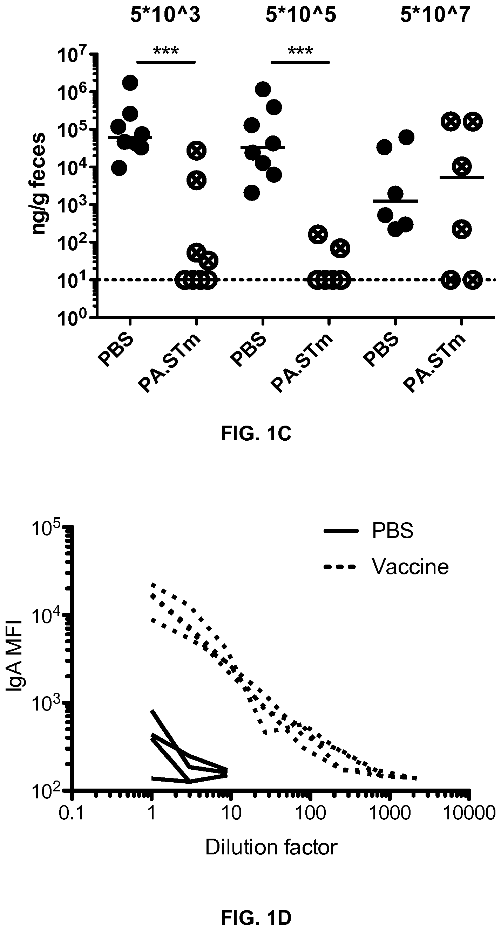

- FIG. 1C:: Graph shows the amount of Lipocalin2 in feces (ng/g) of mice that were either vaccinated with 1e10 particles of peracetic acid inactivated wild-type *S. Typhimurium* strain SL1344 (PA.STm) or vehicle-only control (PBS). The three test groups of mice were orally challenged with live wild-type S. Typhimurium strain SL1344 with an inoculum size of 5^{∗}10³, 5^{∗}10⁵, and 5^{∗}10⁷. The graph demonstrates that inflammation was reduced but still present in wild-type *Salmonella* Typhimurium vaccine treated mice.
- FIG. 1D:: Graph shows the log-median fluorescence intensities (MFI) plotted against antibody serial dilutions for each sample from vaccinated mouse and a mouse treated with PBS. From the vaccinated mice, an intestinal lavage was collected. For both the vaccinated (dotted line) and the PBS (straight black line) treated mice, the intestinal supernatants were used to perform serial dilutions. 25µl of the dilutions (containing intestinal IgA) were incubated with 5^{∗}10⁵ S. Typhimurium. The bacteria were washed and a secondary, fluorescently-labelled anti-IgA antibody. The fluorescence per *S.* Typhimurium cell quantifies the bound IgA and was determined by bacterial flow cytometry. This experiment revealed that all vaccinated mice had robust IgA responses against the wild-type *S.* Typhimurium, i.e. that vaccine failure was not due to failure to induce an intestinal IgA response.

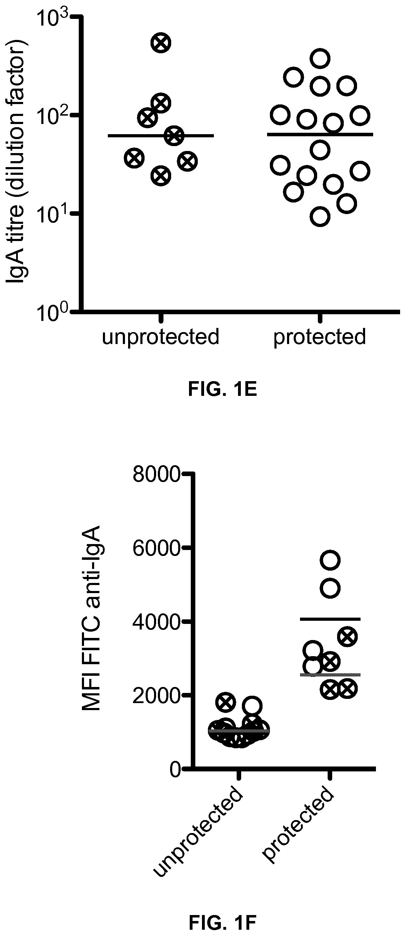

- FIG. 1E:: Graph shows the vaccine-specific intestinal IgA titre production in mice that were vaccinated but developed intestinal inflammation and invasive disease shown in circles with crosses (vaccinated) (unprotected) and in mice that were vaccinated but did not develop intestinal inflammation and invasive disease shown in white circles (vaccinated) (protected). This experiment revealed that all mice had equally robust IgA responses against wild-type *S.* Typhimurium, despite mouse pathology.
- FIG. 1F:: Graph shows the log-median fluorescence intensities (MFI) of intestinal IgA bound to *S.* Typhimurium clones that were isolated from mice that were vaccinated but developed intestinal inflammation and invasive disease shown in circles with crosses (unprotected) and from *S.* Typhimurium clones that were isolated from mice that were vaccinated but did not develop intestinal inflammation and invasive disease shown in white circles (protected). This revealed that *S*.Typhimurium in the intestines of these vaccinated but unprotected mice no longer bound vaccine-induced IgA - i.e. *S.* Typhimurium can escape the IgA response induced by a standard oral vaccine.

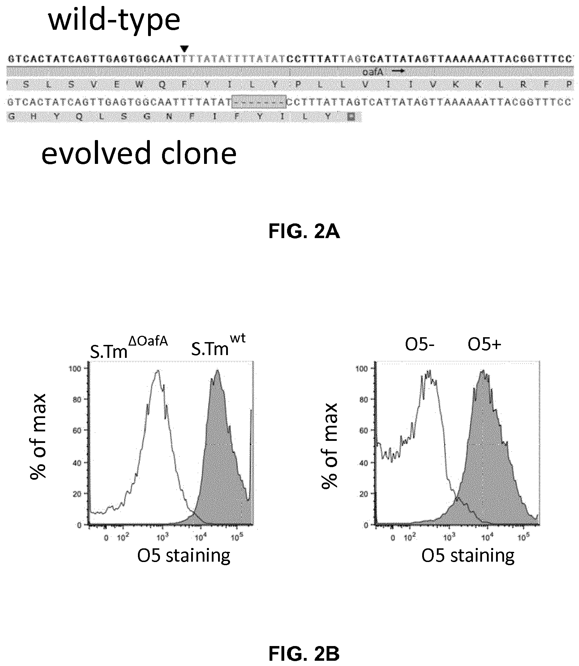

- FIG. 2A:: Genome sequencing of *S.* Typhimurium clones that were isolated from mice that were vaccinated but developed intestinal inflammation and invasive disease (unprotected). The sequencing revealed a common 7 base-pair contraction in the coding region of the OafA gene, known to encode an Abequose O-acetyl transferase which modifies the *S. Typhimurium* O-antigen glycan repeat, the top sequence is the wild type *S. Typhimurium* nucleic acid sequence (SEQ ID NO: 1) and amino acid sequence (SEQ ID NO: 45) and the evolved clone is at the bottom with the comparable nucleic acid sequence (SEQ ID NO: 2) and amino acid sequence (SEQ ID NO:46).
- FIG 2B:: Clones of bacteria that were isolated from mice that were vaccinated but did develop intestinal inflammation and invasive disease (O5-, O5+) were stained with anti-O5 antisera and secondary antibody fluorescent anti-rabbit IgG and compared with *S.* Typhimurium wild type stained bacteria (S.Tm^{WT}). The first graph shows O5 staining of the clean deletion mutant of *OafA* (S.Tm^{ΔOafA}) and for wild type S. Typhimurium (S.Tm^{WT}). The second graph shows staining of two evolved *S*.Typhimurium clones reisolated from infected, vaccinated, but unprotected mice, stained for O5. The graphs show that some *S*.Typhimurium clones from vaccinated but unprotected mice had a loss of O5 compared to wild type S. Typhimurium stained clones (S.Tm^{WT}), and this was phenotypically identical to deletion of the *OafA* gene.

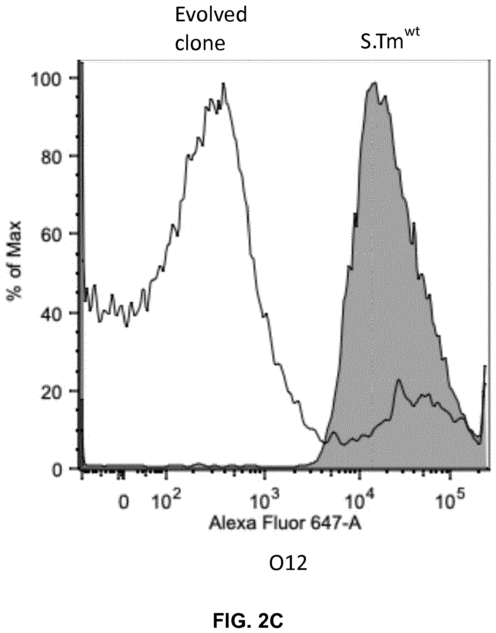

- FIG 2C:: Clones of bacteria that were isolated from mice that were vaccinated but did develop intestinal inflammation and invasive disease (Evolved clone) were stained with anti-O12 antibody and secondary antibody Alexa 647-anti-human IgG and compared with S. Typhimurim wild type stained bacteria (S.Tm^{WT}). This identified a second class of clones with apparently bi-stable loss of binding to an anti-O12 typing antibody. The Y-axis represents the % MFI as determined by bacterial flow cytometry.

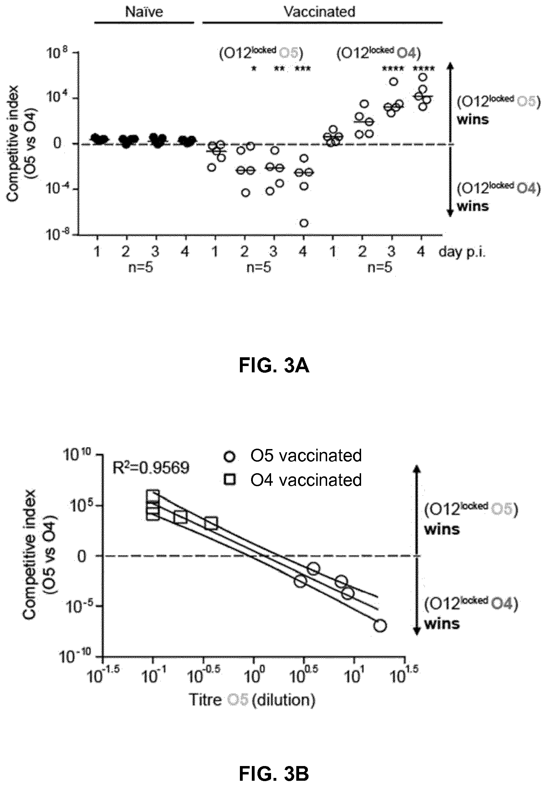

- FIG. 3A:: Competitive infections assay between mice (n=5) vaccinated with vaccines of O5 serovar (acetylated O-antigen) *S.* Typhimurium (O12^{locked}O5) (PA-S.Tm^{ΔgtrC}) or mice (n=5) vaccinated with *S.* Typhimurium carrying an in-frame deletion of OafA (non-acetylated O-antigen, O4 serovar) (O12^{locked}O4) (PA-S.Tm^{ΔoafAΔgtrC}) or mice (n=5) not vaccinated (Naive) and all were subsequently challenged with a 1:1 ratio of O12^{locked}O5 and O12^{locked}O4 S. Typhimurium. The Y-axis measures the ratio of O5 S. Typhimurium to O4 *S.* Typhimurium in feces and the x-axis is the days post infection. The graph shows that in naive mice, both serovars remained at a 1:1 ratio in intestinal content. Mice vaccinated with the inactivated O4 serovar (S.Tm^{ΔoafAΔgtrC}) had an up to 10⁸-fold over-abundance of the O5 serovar (S.Tm^{ΔgtrC}) over the O4 serovar and mice vaccinated with the inactivated O5 serovar had an up to 10⁸-fold over abundance of the O4 serovar over the O5 serovar. The graph demonstrates a very strong selective pressure exerted on the *Salmonella* Typhimurium O-antigen by vaccine-induced IgA. Black circles represent S. typhimurium of naive vaccinated mice and white circles represent S. typhimurium from vaccinated mice.
- FIG 3B:: The intestinal IgA titre specific for the O5 serovar of S.Typhimurium was determined by flow cytometry and plotted against the day 4 competitive index (as in FIG 3A). The graph demonstrates a very strong correlation between the strength of the specific IgA response and the selective pressure on the *Salmonella* Typhimurium O-antigen by vaccine-induced IgA. White circles represent IgA antibodies from O5 (PA-S.Tm^{ΔgtrC}) vaccinated mice and white squares represent IgA antibodies from O4 (PA-S.Tm^{ΔoafAΔgtrC}) vaccinated mice.

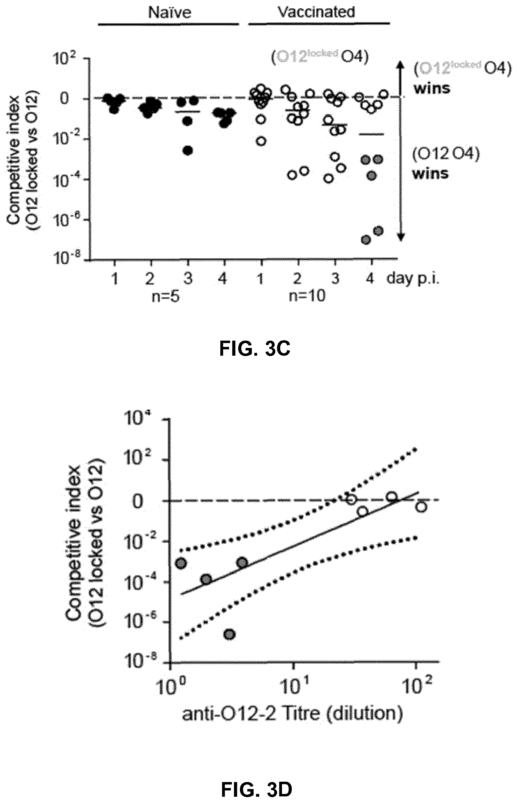

- FIG 3C:: Competitive infections assay between mice (n=10) vaccinated with *S.* Typhimurium carrying in frame deletions of GtrC and OafA (non-acetylated O-antigen, cannot glucosylate, O4,O12^{locked} serovar) (PA-S.Tm^{ΔoafAΔgtrC}) or mice (n=5) not vaccinated (Naive) and all were subsequently challenged with a 1:1 ratio of S. Typhimurium that has a non-acetylated O-antigen and which cannot glucosylate (O12^{locked}O4) (S.Tm^{ΔoafAΔgtrC}) and Serovar 012 O4 (S.Tm^{ΔoafA}) which can glucosylate the O-antigen. Black circles are naive mice. White circles are vaccinated mice that did not develop intestinal inflammation and invasive disease (protected). Grey circles are mice that were vaccinated but developed intestinal inflammation and invasive disease (unprotected). The Y-axis measures the ratio of O12^{locked} to 012 *S*.Typhimurium in feces and the x-axis is the days post infection. In Naive mice both 01204 and O12^{locked}O4 survive in equal measure. In vaccinated mice O12^{locked}O4 dominates in 50% of animals. The assay demonstrated the key role of bistable glucosylation of the O-antigen in escape of IgA-mediated recognition of these bacteria.
- FIG. 3D:: The intestinal IgA titre specific for the 012-2 (glucosylated) serovar of S. Typhimurium was determined by flow cytometry and plotted against the day 4 competitive index in feces (as in FIG 3C). The graph demonstrates a correlation between the strength of the specific IgA response and the selective pressure on the *Salmonella* Typhimurium O-antigen by vaccine-induced IgA. White circles are vaccinated mice and did not develop intestinal inflammation and invasive disease (protected). Grey circles are mice that were vaccinated but developed intestinal inflammation and invasive disease (unprotected). The Y-axis measures S. Typhimurium competitive index in feces (ratio of O12^{locked} to 012 *S*.Typhimurium in feces at d4 post-infection) and the x-axis measures the anti-O12^{locked}O4 antibody titre (anti-O12-2 Ab). Out competition of O12-locked strains was only observed in mice with weak 012-2 antibody response.

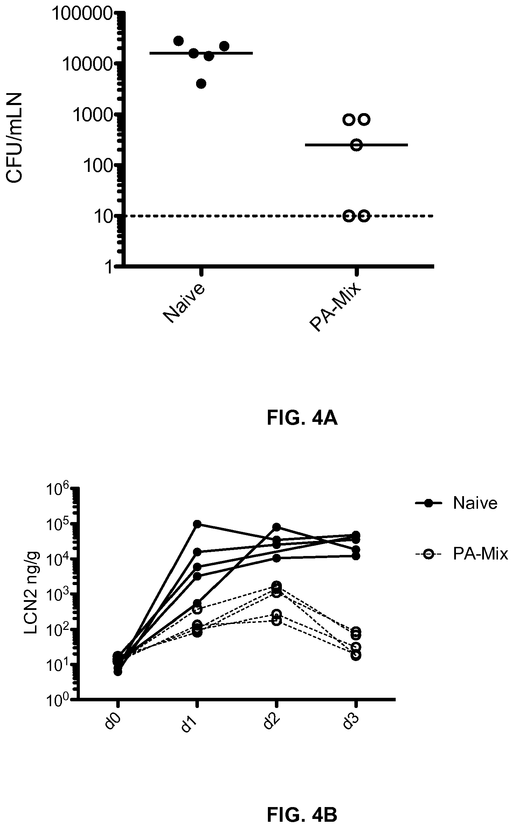

- FIG. 4A:: Graph shows the *Salmonella* Colony Forming Units per mesenteric lymph node (CFU/mLN) of mice which that were either vaccinated with a 1:1:1:1 mix of 1e10 particles of peracetic acid inactivated *S. Typhimurium* strains: Serovar O5, 012, Serovar O4, 012, Serovar O5, 012-2 and Serovar O4, 012-2 (PA-Mix) or vaccinated with a vehicle-only control PBS (Naive). The mice were orally challenged with live wild-type S. Typhimurium strain SL1344 with an inoculum size of 1e5. Black circles are unvaccinated (naive) mice and white circles are vaccinated (PA-Mix) mice. The graph demonstrates that an Evolutionary Trap vaccine (PA-Mix) constructed by combining escape mutants (the four serovar), generated robust vaccine-mediated protection.
- FIG. 4B:: Graph shows the amount of Lipocalin2 in feces (ng/g) of mice that were either vaccinated with a 1:1:1:1 mix of 1e10 particles of peracetic acid inactivated *S. Typhimurium* strains: Serovar O5, 012, Serovar O4, 012, Serovar O5, 012-2 and Serovar O4, 012-2 (PA-Mix) or vaccinated with vehicle-only control PBS (Naive). The mice were orally challenged with live wild-type S. Typhimurium strain SL1344 with an inoculum size of 1e5. Black circles are unvaccinated (naive) mice and white circles are vaccinated (PA-Mix) mice. The X-axis is days post infection. The graph shows intestinal inflammation in naive mice versus mice that were vaccinated. The graph demonstrates that an Evolutionary Trap vaccine (PA-Mix), constructed by combining escape mutants (the four serovar), generated robust vaccine-mediated protection from inflammatory disease.

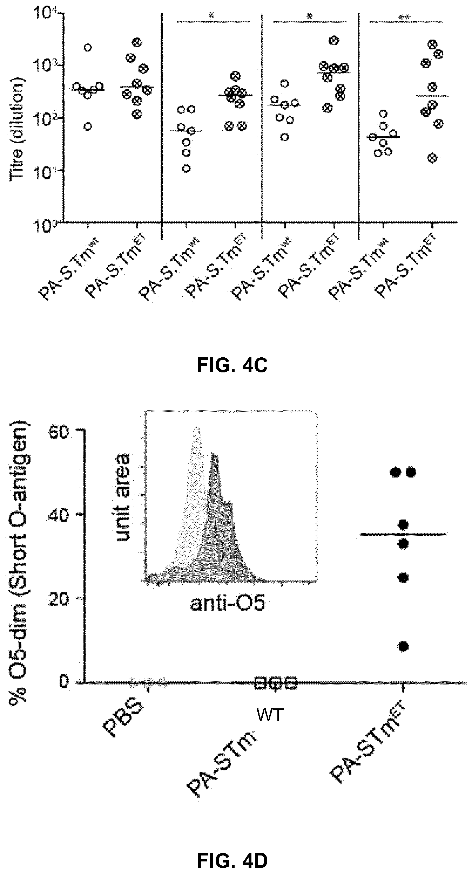

- FIG. 4C:: Graph shows the amount of antibody produced in mice which that were either vaccinated with a 1:1:1:1 mix of 1e10 particles of peracetic acid inactivated *S. Typhimurium* strains: Serovar O5, 012, Serovar O4, 012, Serovar O5, 012-2 and Serovar O4, 012-2 (PA-S.Tm^{ET}) or vaccinated with wild type S. Typhimurium (PA-S.Tm^{WT}). White circles are mice vaccinated with wild type S. Typhimurium (PA-S.Tm^{WT}) and circles with black crosses are mice vaccinated with PA-S.Tm^{ET}. At 28 days after the first vaccination, mice receiving the combined vaccine had significantly higher intestinal IgA antibody titres with specificities towards all three serovar variants: O4/O12 (second column), O5/O12-2 (third column) and O4/O12-2 (fourth column) compared to wild type S. Typhimurium vaccinated mice. The first column was IgA antibody titres with specificities towards O5, 012.
- FIG. 4D:: Graph shows the fraction of *S*.Typhimurium clones with a short O-antigen recovered from mice that were either vaccinated with a 1:1:1:1 mix of 1e10 particles of peracetic acid inactivated *S. Typhimurium* strains: Serovar O5, 012, Serovar O4, 012, Serovar O5, 012-2 and Serovar O4, 012-2 (PA-S.Tm^{ET}) or vaccinated with wild type S. Typhimurium (PA-S.Tm^{WT}) or in unvaccinated mice (PBS). The mice were orally challenged with live wild-type *S.* Typhimurium strain SL1344 with an inoculum size of 1e5. White squares are vaccinated with wild type S. Typhimurium (PA-S.Tm^{WT}) mice and black circles are vaccinated with PA-S.Tm^{ET} mice. At 4 days post challenge the salmonella clones from the mouse gut were detected by dim staining with the anti-O5 antibody by flow cytometry (% O5-dim - inset graph). Mice receiving the combined vaccine had a significantly higher fraction of S. Typhimurium with short O-antigens compared to wild type vaccinated and PBS treated mice.

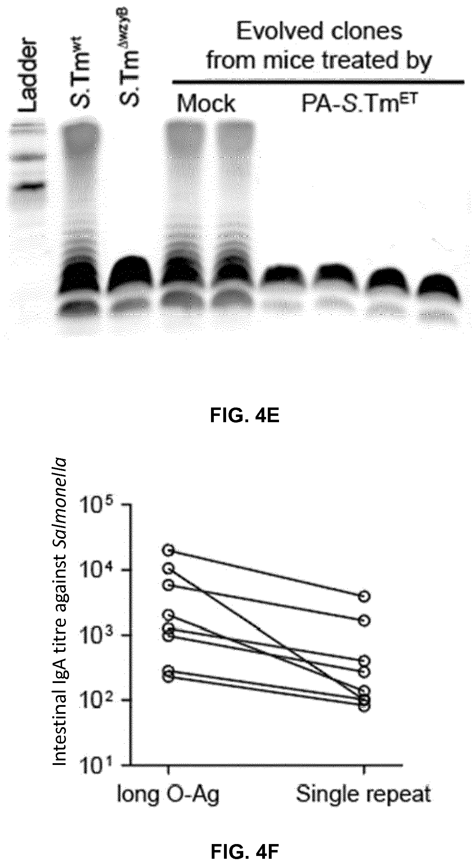

- FIG. 4E:: Gel shows the short O-antigen of S. Typhimurium recovered from mice that were either vaccinated with a 1:1:1:1 mix of 1e10 particles of peracetic acid inactivated *S. Typhimurium* strains: Serovar O5, 012, Serovar O4, 012, Serovar O5, 012-2 and Serovar O4, 012-2 (PA-S.Tm^{ET}) or vaccinated with wild type S. Typhimurium (PA-S.Tm^{WT}) or in unvaccinated mice (PBS). The mice were orally challenged with live wild-type S. Typhimurium strain SL1344 with an inoculum size of 1e5. At 4 days post challenge the salmonella clones from the mouse gut were measured for the short O-antigen. The gel contains five control columns, a ladder, a positive control S. Typhimurium with a constructed short O-antigen (S.Tm^{wzyB}), clones from wild type S. typhimurium vaccinated mice (S.Tm^{WT}), clones from unvaccinated mice (Mock). The last four columns are clones from mice vaccinated with wild type S. Typhimurium (PA-S.Tm^{ET}). The clones from mice that received the combined vaccine PA-S.Tm^{ET} contained S.Typhimurium clones with short O-antigens compared to wild type vaccinated and PBS treated mice.
- FIG. 4F:: This graph shows intestinal IgA antibodies from mice which were vaccinated with a 1:1:1:1 mix of 1e10 particles of peracetic acid inactivated *S. Typhimurium* strains: Serovar O5, 012, Serovar O4, 012, Serovar O5, 012-2 and Serovar O4, 012-2 (PA-S.Tm^{ET}). For each intestinal IgA sample, we quantified the ability to bind to S. Typhimurium with either a short O-antigen (S.Tm^{wzyB}) (Single repeat) or the wild type S. Typhimurium (long O-Ag). The single repeat O-antigen resulted in lower binding to vaccine-induced IgA, explaining the emergence of these clones in vaccinated mice.

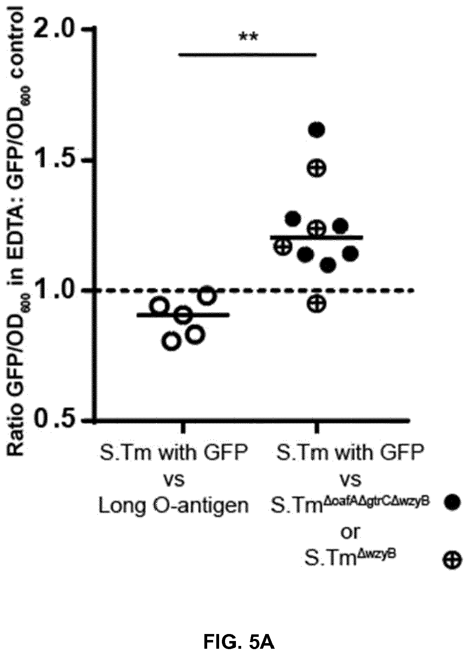

- FIG. 5A:: The graph shows the mean ratio of GFP signal to OD (fluorescence intensity) measured during the last 100 minutes of incubation of bacteria under stressful conditions (Tris and EDTA), this measured the relative survival of wild-type (expressing GFP) and test bacteria (contributing only to OD) following stress testing. OD and fluorescence values were corrected for the baseline value measured at time 0. The Y-axis is the OD ratio of GFP. The first column measures cell death via OD of S. Typhimurium producing full length antigen (long O antigen) (S.Tm^{ΔoafAΔgtrC}) versus a wild type S. Typhimurium with GFP (S.Tm^{GFP}). The second column black circles measure cell death via OD of S. Typhimurium with OAFA GTRC mutant with in-frame deletion mutant for wzyB (S.Tm^{ΔoafAΔgtrCΔwzyB}) (short O-antigen) versus a wild type S. Typhimurium with GFP (S.Tm^{GFP}). The second column cross circles measure cell death via OD of an evolved S. Typhimurium clone from a vaccinated mouse which carries a genomic deletion including the wzyB gene (S.Tm^{ΔwzyB}) (short O antigen) versus a wild type S. Typhimurium with GFP (S.Tm^{GFP}). Graph demonstrates fitness defects of the short O-antigen mutants and lower survival rate following stressful conditions (Tris and EDTA).

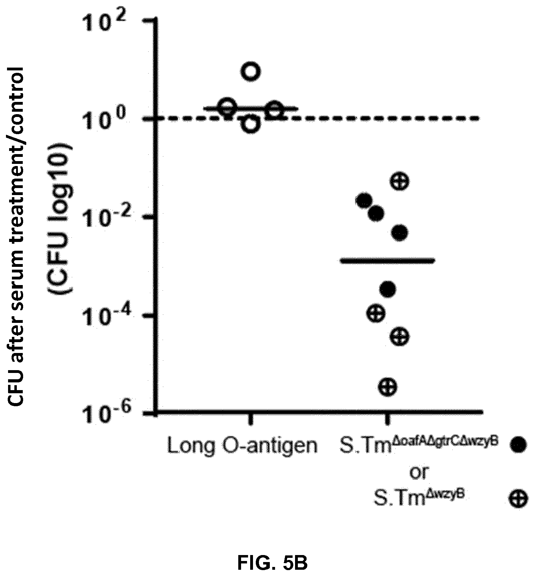

- FIG 5B:: The graph shows the colony forming units (CFU log10) measured 1h after incubation of the bacteria with human serum, this measured the survival of the bacteria following incubation with human complement proteins. The Y-axis is the colony forming units (CFU log10). The first column measures bacteria count (survival) of S. Typhimurium with OAFA GTRC mutant producing full length antigen (long O-antigen) (S.Tm^{ΔoafAΔgtrC}). The second column black circles measure bacteria count (survival) of S. Typhimurium with OAFA GTRC mutant with in-frame deletion mutant for wzyB (S.Tm^{ΔoafAΔgtrCΔwzyB}) (short O-antigen). The second column cross circles measure bacteria count (survival) of evolved S. Typhimurium clone from a vaccinated mouse which carries a genomic deletion including the wzyB gene (S.Tm^{ΔwzyB}) (short O-antigen). Graph demonstrates fitness defects of the short O-antigen mutants and lower survival rate following incubation with human serum (complement proteins).

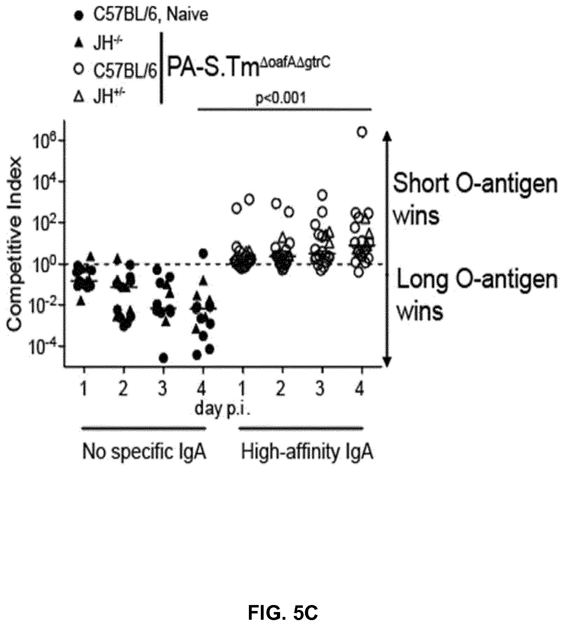

- FIG 5C:: A mouse model which cannot produce antibodies (JH-/-), two mouse models that can produce antibodies (JH+/- and C57BL/6) were vaccinated with S. Typhimurium with OAFA GTRC mutant (S.Tm^{ΔoafAΔgtrC}) (long, non-acetylated, no glucosylated O-antigen). A mouse model that can produce antibodies (C57BL/6) was unvaccinated (naive). All mice were challenged with S. Typhimurium with a 1:1 ratio of OAFA GTRC mutant producing full length antigen (long O antigen) (S.Tm^{ΔoafAΔgtrC}) and S. Typhimurium with OAFA GTRC mutant with in-frame deletion mutant for wzyB (S.Tm^{ΔoafAΔgtrCΔwzyB}) (short O-antigen). The Y-axis measures the ratio of s.Tm^{ΔoafAΔgtrCΔwzyB} to s.Tm^{ΔoafAΔgtrC} in feces. The X-axis is the days post infection. Black triangles are JH-/- vaccinated mice (no antibodies, control for non-immune effects of vaccination) and black circles are C57BL/6 unvaccinated mice, in both cases the long O-antigen bacteria dominated (S.Tm^{ΔoafAΔgtrC}). White Circles are C57BL/6 vaccinated mice and white triangles are JH+/- vaccinated mice (specific IgA antibodies), in both cases the short O-antigen bacteria dominated (S.Tm^{ΔoafAΔgtrCΔwzyB}). This demonstrates that specific IgA can strongly select for the evolution of S.Typhimurium carrying deletions in wzyB, and therefore with a short O-antigen.

### DETAILED DESCRIPTION OF THE INVENTION

Unless defined otherwise, all technical and scientific terms used herein have the same meanings as commonly understood by one of ordinary skill in the art to which this invention belongs.

Specific Proteobacterial O-antigen modifications can be used to generate vaccines that induce antibodies against the typical serovar-associated O-antigen, and against all escape variants. We discovered 1) that escape variants can be identified by iterative vaccination and infection in experimental settings and 2) that inducing antibodies against all naturally-arising escape variants is not only critical to make a non-escapable vaccine but also drives evolution of the Proteobacterial target into a dead-end, preventing efficient transmission, i.e. oral vaccines can be used to rationally control bacterial pathogen evolution. This has been specifically demonstrated in *Salmonella enterica* subspecies *enterica* serovar Typhimurium. Vaccine escape variants were found to carry deletions or epigenetic modifications of O-antigen modifying genes, generating chemical modifications (acetylation, glucosylation) of the O-antigen glycan repeating unit. Rationally combining strains engineered to produce all possible modified O-antigen structures, generates a vaccine which increased the strength of protection per se over the standard wild-type vaccine. Surprisingly vaccination with a combination of these vaccines forces the emergence of a further type of O-antigen variation: production of single-repeat (i.e. very short) O-antigen. Mice which had received the combined vaccine, produced *S. Typhimurium* carrying a spontaneous deletion of the O-antigen polymerase (therefore producing very short O-antigens) which outgrew and replaced the wild type *S. Typhimurium* cells in the gut. However, this population of *S. Typhimurium* with very short O-antigen has a dramatic loss of fitness when these strains are transmitted into naive hosts, and during environmental phases of the transmission cycle. This breaks the transmission chain of the pathogen in the host population, effectively forcing bacterial evolution into a dead-end. The theory underlining vaccine design to drive pathogens into evolutionary dead ends can be applied to all Proteobacteria as they contain similar polymerized O-antigens and the process of selecting for bacterial escape variants will be identical. The theory underlining vaccine design to drive pathogens into evolutionary dead ends can be applied to all Gammaproteobacteria as they also contain similar polymerized O-antigens and the process of selecting for bacterial escape variants will be identical.

Several aspects of the present invention are disclosed herein; the embodiments and preferred embodiments, respectively, mentioned further herein are applicable for each and any of the 8 or further aspects of the present invention disclosed herein, even though not explicitly mentioned.

A first aspect of the present invention is an immunogenic composition comprising at least two or more inactivated serovar of a Proteobacteria strain, wherein each of said two or more inactivated serovar comprises a genetic modification of the O-antigen, wherein independently each of said genetic modification comprises a glucosylation and/or O-acetylation of the O-antigen. Thus, each of said two or more inactivated serovar comprises a genetic modification that alters the O-antigen.

In one embodiment, the genetic modification of the O-antigen is a glucosylation of the O-antigen. In another embodiment of the present invention, the genetic modification of the O-antigen is an O-acetylation of the O-antigen. Thus, the genetic modification that alters the O-antigen is a glucosylation of the O-antigen or a genetic modification that modifies the O-antigen is an O-acetylation of the O-antigen.

In one embodiment, the composition additionally comprises the inactivated wild type serovar of said Proteobacteria strain.

In another embodiment, the genetic modification of the O-antigen are created following deletions and/or epigenetic modifications of the O-antigen modifying genes Abequose O-acetyl transferase (OafA gene) and gtrABC operons (gtrC gene).

In another embodiment, the genetic modification of the O-antigen is created following deletions and/or epigenetic modifications of the O-antigen modifying genes of said inactivated serovar. In one embodiment, the O-antigen modifying genes comprise Abequose O-acetyl transferase (OafA gene) and gtrABC operons (gtrC gene). Thus, the genetic modification that alters the O-antigen are created following deletions and/or epigenetic modifications of the O-antigen modifying genes Abequose O-acetyl transferase (OafA gene) and gtrABC operons (gtrC gene).

In another embodiment, the composition induces in an animal, upon infection with the wild type serovar of said Proteobacteria, the production of a serovar of said Proteobacteria strain with decreased virulence, compared to animals not exposed to the immunogenic composition, wherein decreased virulence is measured as a decrease in mortality and/or morbidity of the infected animal.

"Decreased virulence" in a bacterium refers to altering expression of genes associated with virulence, including regulators of virulence. Decreased virulence also refers to physical and biochemical manifestations of virulence including those manifestations associated with any step of the bacterial life cycle when it is associated with a host, including without limitation the adherence, invasion, replication, evasion of host defenses, and transmittal to a new host. Decreased bacterial virulence may be manifested in the form of reduced symptoms in a host, and thus may be detected by monitoring the host for a reduced reaction to the bacteria associated therewith. A decrease in virulence may be at least about a 1 % reduction, at least about a 10% reduction, at least about a 20% reduction, at least about a 30% reduction, at least about a 40% reduction, at least about a 50% reduction, at least about a 60% reduction, at least about a 70% reduction, at least about a 80% reduction, at least about a 90% reduction, or at least about a 100% reduction of virulence, as measured by any assay known to those of skilled in the art, when measured against a suitable control.

In another embodiment, the composition induces in an animal, upon infection with the wild type serovar of said proteobacteria, the production of a serovar of said proteobacteria strain with a decreased transmission rate, compared to animals not exposed to the immunogenic composition.

"Transmission rate" in a bacterium refers to the disease incidence or the number of infected cases. A decrease in transmission rate may be manifested in the form of a reduction or no symptoms in a host, and thus may be detected by monitoring the host for a reduced reaction to the bacteria associated therewith or may be detected by monitoring the level of said bacterial infection or presence of said bacteria in the host. A decrease in transmission rate may be at least about a 1 % reduction, at least about a 10% reduction, at least about a 20% reduction, at least about a 30% reduction, at least about a 40% reduction, at least about a 50% reduction, at least about a 60% reduction, at least about a 70% reduction, at least about a 80% reduction, at least about a 90% reduction, or at least about a 100% reduction of transmission, as measured by any assay known to those of skilled in the art, when measured against a suitable control.

In another embodiment, the proteobacteria is *Salmonella spp* or *Escherichia coli.* In another embodiment, the proteobacteria is *Salmonella spp.* In another embodiment of the first aspect of the present invention, the proteobacteria is *Escherichia coli.*

In another embodiment, the proteobacteria is *Salmonella enterica,* and wherein preferably said *Salmonella enterica is Salmonella enterica subspecies enterica.* In another embodiment, the proteobacteria is *Salmonella enterica subspecies enterica.*

In another embodiment, the proteobacteria is *Salmonella enterica subspecies enterica,* wherein said *Salmonella enterica subspecies enterica* is selected from the group consisting of: *Typhi, Paratyphi A Paratyphi B, Typhimurium, Montevideo, Gallinarium, Anatum, Pullorum, Muenchen, Kentucky, Dublin, Derby, Minnesota and Enteritidis.* In another embodiment, the proteobacteria is *Salmonella enterica subspecies enterica,* wherein said *Salmonella enterica subspecies enterica* is a serovar selected from the group of consisting of: *Typhi, Paratyphi A Paratyphi B, Typhimurium, Montevideo, Gallinarium, Anatum, Pullorum, Muenchen, Kentucky, Dublin, Derby, Minnesota and Enteritidis.*

In another embodiment, the proteobacteria is *Salmonella enterica,* wherein said *Salmonella enterica* is *Salmonella enterica subspecies enterica* serovar Typhimurium.

In another embodiment, the proteobacteria is *Salmonella enterica subspecies enterica* serovar Typhimurium *strain* ATCC SL1344 or ATCC 14028. In another embodiment, the proteobacteria is *Salmonella enterica subspecies enterica* serovar Typhimurium *strain* ATCC SL1344. In another embodiment, the proteobacteria is *Salmonella enterica subspecies enterica* serovar Typhimurium *strain* ATCC 14028. *Salmonella enterica serovar* *Typhimurium* strain ATCC SL1344 or ATCC 14028 are both available from the American Type Culture Collection. It is a common assumption that data obtained with one strain is representative of both the Typhimurium serovar and the *S. enterica* species as a whole.

*Salmonella* as used herein refers to any strain of *Salmonella,* including any strain of *Salmonella enterica,* including *Salmonella enterica serovar Typhimurium.* The serovars of *S. enterica* that may be used as the attenuated bacterium of the live compositions described in accordance with various embodiments herein include, without limitation, *Salmonella enterica serovar Typhimurium, Salmonella montevideo, Salmonella enterica serovar Typhi, Salmonella enterica serovar, Paratyphi B, Salmonella enterica serovar Paratyphi C, Salmonella enterica serovar Hadar, Salmonella enterica serovar Enteriditis, Salmonella enterica serovar Kentucky, Salmonella enterica serovar In*/*antis, Salmonella enterica serovar Pullorurn, Salmonella enterica serovar Gallinarum, Salmonella enterica serovar Muenchen, Salmonella enterica serovar Anaturn, Salmonella enterica serovar Dublin, Salmonella enterica serovar Derby, Salmonella enterica serovar Choleraesuis var. kunzendorf and Salmonella enterica serovar minnesota,* among other known strains.

As used herein, an immunogenic composition is typically and preferably a composition to which a humoral (e.g., antibody) or cellular (e.g., a cytotoxic T cell) response, or, in one embodiment, an innate immune response, is mounted following delivery of said immunogenic composition to a mammal or animal subject.

The term "inactivated" refers to a previously virulent or non-virulent bacteria or bacterium that has been irradiated (ultraviolet (UV), X-ray, electron beam or gamma radiation), heated, or chemically treated to inactivate or kill said bacteria or bacterium, while retaining its immunogenicity. In one embodiment, the inactivated bacteria disclosed herein are inactivated by treatment with an inactivating agent. Suitable inactivating agents include beta-propiolactone, binary or beta- or acetyl-ethyleneimine, glutaraldehyde, ozone, peracetic acid and Formalin (formaldehyde). For inactivation by formalin or formaldehyde, formaldehyde is typically mixed with water and methyl alcohol to create formalin. In a preferred embodiment, the inactivating agent is peracetic acid. More preferably, the inactivating agent is peracetic acid and it is used in the range of a final concentration with the bacteria, of between 0.5% - 20%, 0.5% - 10%, 0.2%-5%, 0.5%-4% or 1%-2%.

More particularly, the term "inactivated" in the context of a bacteria means that the bacteria is incapable of replication *in vivo* or *in vitro* and, respectively, the term "inactivated" in the context of a bacteria means that the bacteria is incapable of reproduction *in vivo* or *in vitro.* For example, the term "inactivated" may refer, in an embodiment, to a bacteria that has been propagated, typically and preferably *in vitro,* and has then been inactivated using chemical or physical means so that it is no longer capable of replicating.

Even more particularly, the term "inactivated" in the context of the present invention means that the bacteria is inactivated by treatment with an inactivating agent, wherein said inactivating agent is preferably peracetic acid, in the manner as described in Example 1 and the referred Moor et al Frontiers in Immunology, (2016) Vol 7, Article 34.

The following disclosure of the immunogenic compositions and methods of this invention specifically describes vaccine compositions for prophylactic use against Proteobacteria. The term "vaccine", as used herein, is a substance used to stimulate the immune response and provide immunity against one or several diseases.

The term "wild type *Salmonella",* as used herein, is in particular directed to an infectious pathogenic *Salmonella,* which is particularly capable of infection in swine.

"Recombinant", as applied to a polynucleotide, means that the polynucleotide is the product of various combinations of cloning, restriction or ligation steps, and other procedures that result in a construct that is distinct from a polynucleotide found in nature. A recombinant bacterium is a bacterial cell comprising a recombinant polynucleotide. The terms respectively include replicates of the original polynucleotide construct and progeny of the original bacterial construct. Typical recombinant or genetic engineering steps to generate a recombinant bacterium as referred to herein include bacteriophage transduction or homologous recombination, among other known techniques described in the art.

In another embodiment of the immunogenic composition as described herein, the inactivated serovar may be attenuated prior to or after to genetic modification of O-Antigen.

The terms "modification" refers to any deliberately inserted change in a nucleic acid or protein sequence, such as a deletion of all or part of the O-antigen sequences, or an insertion of a sequence into the O-antigen sequences.

"Naturally occurring" means a sequence found in nature and not synthetically prepared or modified.

In another embodiment of the immunogenic composition of the present invention, the inactivated serovar are produced via bacteriophage, transduction or homologous recombination.

Deletion or insertion in genes involved in determining the O-antigen structure may be engineered using a conventional technique, such as homologous recombination, transposon insertion or bacteriophage transduction. All of these techniques are known in the art.

In another embodiment of the present invention, said O-antigens of said *Salmonella enterica* serovar Typhimurium (*S*. Tm) comprise a mannose-rhamnose-galactose-repeat with the modification being (i) in the identity of the mannose-linked di-deoxyhexose, i.e. an O-acetylated Abequose residue (O5 serovar) or a non-O-acetylated serotype (O4 serovar) generated by the loss of function of the Abequose O-acetyl transferase OafA, and/or (ii) the galactose residue, i.e. unmodified (012 serovar) or modified by addition of a glucose residue via an α1-4 linkage to the galactose (012-2 serovar).

The structures of the O-antigens of said O5 012 serovar, O4 012 serovar, O5 012-2 serovar and O4, 012-2 serovar are as follows comprising the depicted genetic modifications of said O-antigens oligosaccharide repeats:
O5 O12 serovar: 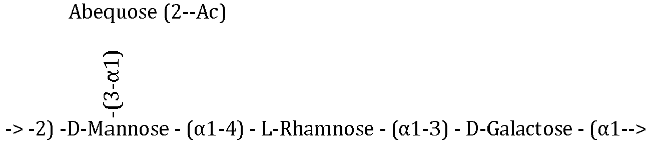
O4 O12 serovar: 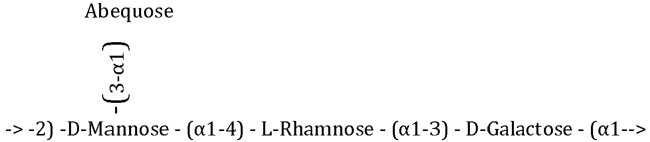
O5 012-2 serovar: 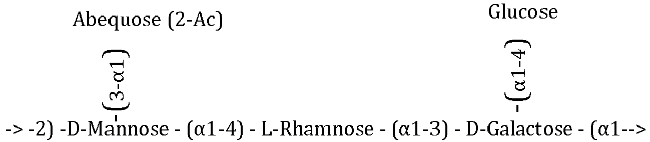 and
O4, 012-2 serovar:

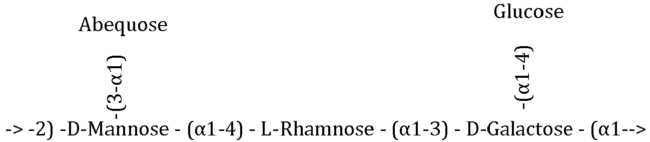

Preferably, the oligosaccharide repeats within the O-antigen between 10-200 times, further preferably 25-150 times. This is symbolized by the arrows flanking the oligosaccharide and glycan structures, respectively.

In another embodiment, the proteobacteria is *Salmonella enterica serovar Typhimurium,* and wherein said at least 2 inactivated serovar of said proteobacteria strain are selected from the group consisting of O5 012 serovar, O4 012 serovar, O5 012-2 serovar and O4, 012-2 serovar, and wherein preferably the O-antigen of said inactivated serovar comprises a glycan structure, typically and preferably oligosaccharide repeats, as depicted above.

In another embodiment of the immunogenic composition, the proteobacteria is *Salmonella enterica subspecies enterica serovar Typhimurium,* and wherein said composition comprises at least 4, preferably exactly 4, inactivated serovar of said proteobacteria strain, and wherein said 4 inactivated serovar are O5 012 serovar, O4 012 serovar, O5 012-2 serovar and O4, 012-2 serovar, and wherein preferably the O-antigen of said four inactivated serovar comprises a glycan structure, typically and preferably oligosaccharide repeats, selected from the following formula:
O5 O12 serovar: 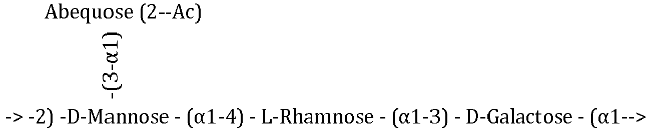
O4 O12 serovar: 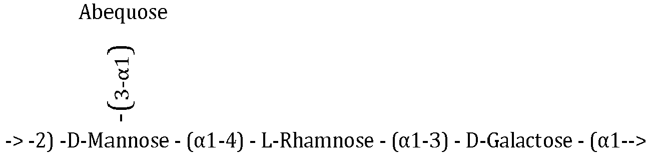
O5 012-2 serovar: 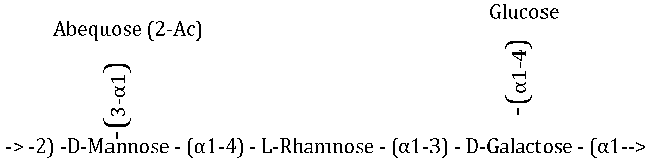 and
O4, 012-2 serovar:

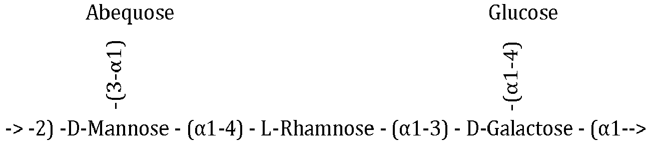

In another embodiment, the proteobacteria is *Salmonella enterica subspecies enterica serovar Typhimurium,* and wherein said composition comprises exactly 4 inactivated serovar of said proteobacteria strain, and wherein said 4 inactivated serovar are O5 012 serovar, O4 012 serovar, O5 012-2 serovar and O4, 012-2 serovar, and wherein preferably the O-antigen of said four inactivated serovar comprises a glycan structure of the following formula:
O5 O12 serovar: 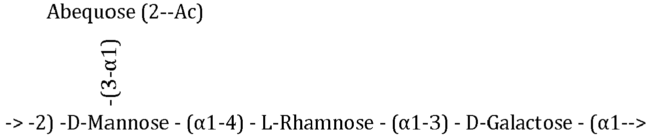
O4 O12 serovar: 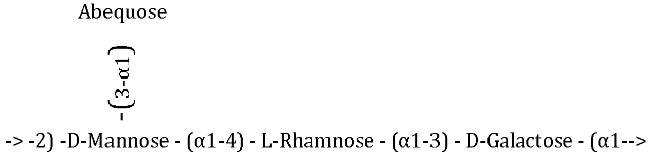
O5 012-2 serovar: 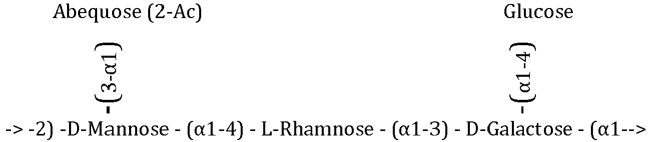 and
O4, 012-2 serovar:

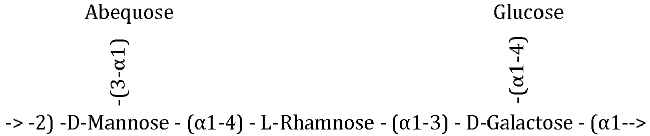

In another embodiment of the immunogenic composition, the proteobacteria is *Salmonella enterica subspecies enterica serovar Typhimurium,* and wherein said composition comprises 2 inactivated serovar O5 012 serovar, O4 012 serovar, and wherein preferably the O-antigen of said two inactivated serovar comprises a glycan structure, preferably oligosaccharide repeats, as depicted above.

In another embodiment of the immunogenic composition, the proteobacteria is *Salmonella enterica subspecies enterica serovar Typhimurium,* and wherein said composition comprises 2 inactivated serovar O5, 012-2 serovar and O4, 012-2 serovar, and wherein preferably the O-antigen of said two inactivated serovar comprises a glycan structure, preferably oligosaccharide repeats, as depicted above.

In another embodiment of the immunogenic composition, the proteobacteria is *Salmonella enterica subspecies enterica serovar Typhimurium,* and wherein said composition comprises 2 inactivated serovar O5 012 serovar, O5 012-2 serovar and wherein preferably the O-antigen of said two inactivated serovar comprises a glycan structure, preferably oligosaccharide repeats, as depicted above.

In another embodiment of the immunogenic composition, the proteobacteria is *Salmonella enterica subspecies enterica serovar Typhimurium,* and wherein said composition comprises 2 inactivated serovar O4 012 serovar and O4, 012-2 serovar, and wherein preferably the O-antigen of said two inactivated serovar comprises a glycan structure, preferably oligosaccharide repeats, as depicted above.

In another embodiment of the immunogenic composition, the proteobacteria is *Salmonella enterica subspecies enterica serovar Typhimurium,* and wherein said composition comprises 2 inactivated serovar are O5 012 serovar, and O4, 012-2 serovar, and wherein preferably the O-antigen of said two inactivated serovar comprises a glycan structure, preferably oligosaccharide repeats, as depicted above.

In another embodiment of the immunogenic composition, the proteobacteria is *Salmonella enterica subspecies enterica serovar Typhimurium,* and wherein said composition comprises 2 inactivated serovar are O4 012 serovar and O5 012-2 serovar and wherein preferably the O-antigen of said two inactivated serovar comprises a glycan structure, preferably oligosaccharide repeats, as depicted above.

In another embodiment there is the immunogenic composition of the first aspect of the present invention, wherein the proteobacteria is *Salmonella enterica subspecies enterica serovar Typhimurium,* and wherein said composition comprises 3 inactivated serovar selected from the group consisting of O5 012 serovar, O4 012 serovar, O5 012-2 serovar and O4, 012-2 serovar, and wherein preferably the O-antigen of said three inactivated serovar comprises a glycan structure, preferably oligosaccharide repeats, as depicted above.

In one embodiment, the O-antigen gene is inserted in the chromosome at a non-naturally occurring site or is present extrachromosomally (plasmid) to enable the proteobacteria to produce or deliver larger amounts of the O-antigen modifying enzymes than would be produced in an unmodified bacterium.

In another embodiment of the immunogenic composition, the proteobacteria is *Salmonella enterica subspecies enterica serovar Typhimurium,* and wherein said composition comprises 3 inactivated serovar, O5 012 serovar, O4 012 serovar, and O5 012-2 serovar and wherein preferably the O-antigen of said three inactivated serovar comprises a glycan structure, preferably oligosaccharide repeats, of the following formula:
O5 O12 serovar: 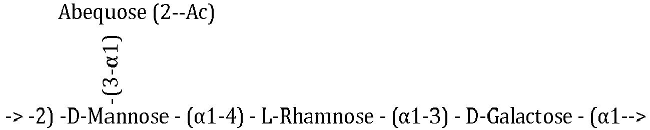
O4 O12 serovar: 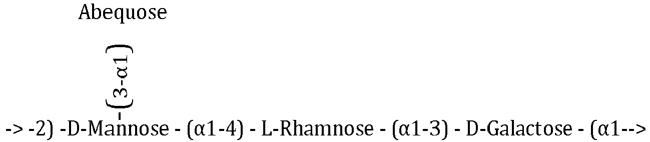 and
O5 012-2 serovar:

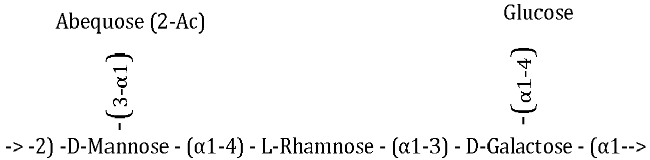

In another embodiment of the immunogenic composition, the proteobacteria is *Salmonella enterica subspecies enterica serovar Typhimurium,* and wherein said composition comprises 3 inactivated serovar, O5 012 serovar, O4 012 serovar and O4, 012-2 serovar, and wherein preferably the O-antigen of said three inactivated serovar comprises a glycan structure, preferably oligosaccharide repeats, of the following formula:
O5 O12 serovar: 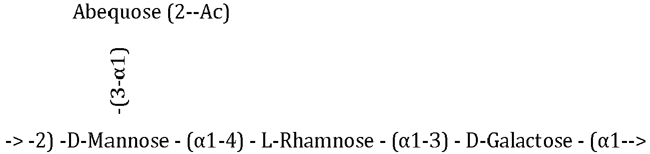
O4 O12 serovar: 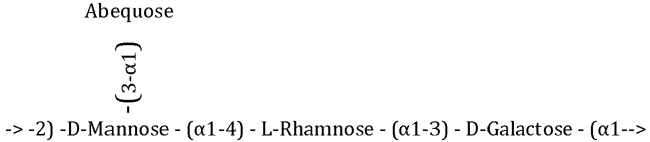 and
O4, 012-2 serovar:

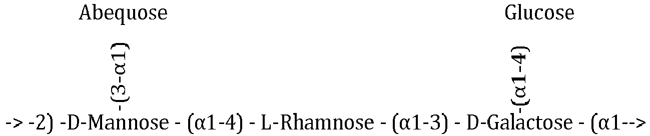

In another embodiment of the immunogenic composition, the proteobacteria is *Salmonella enterica subspecies enterica serovar Typhimurium,* and wherein said composition comprises 3 inactivated serovar are O5 012 serovar, O5 012-2 serovar and O4, 012-2 serovar, and wherein preferably the O-antigen of said three inactivated serovar comprises a glycan structure, preferably oligosaccharide repeats, of the following formula:
O5 O12 serovar: 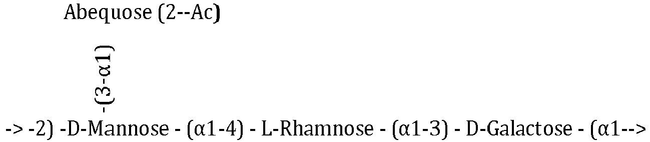
O5 012-2 serovar: 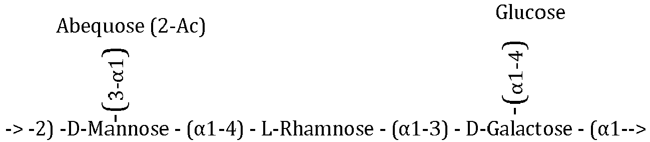 and
O4, 012-2 serovar:

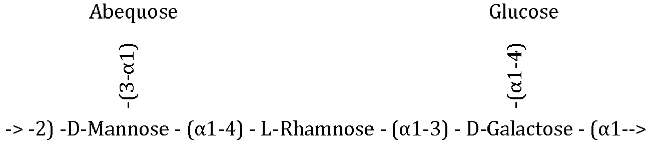

In another embodiment of the immunogenic composition, the proteobacteria is *Salmonella enterica subspecies enterica serovar Typhimurium,* and wherein said composition comprises 3 inactivated serovar O4 012 serovar, O5 012-2 serovar and O4, 012-2 serovar, and wherein preferably the O-antigen of said three inactivated serovar comprises a glycan structure, preferably oligosaccharide repeats, of the following formula:
O4 O12 serovar: 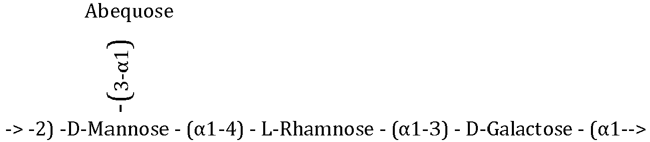
O5 012-2 serovar: 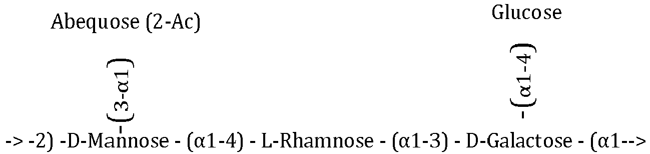 and
O4, 012-2 serovar:

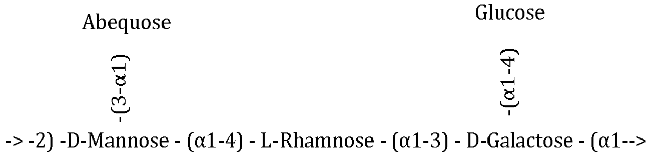

In another embodiment of the immunogenic composition, the proteobacteria is *Salmonella enterica subspecies enterica serovar Typhimurium,* and wherein said composition comprises 3 inactivated serovar are O5 012 serovar, O4 012 serovar and O5 012-2 serovar and wherein preferably the O-antigen of said three inactivated serovar comprises a glycan structure, preferably oligosaccharide repeats, of the following formula:
O5 O12 serovar: 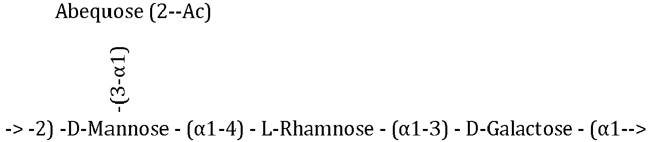
O4 O12 serovar: 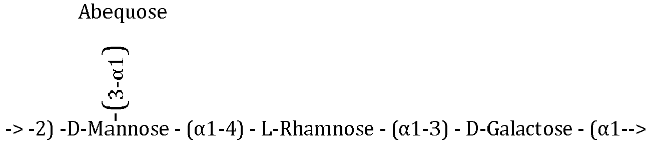 and
O5 012-2 serovar:

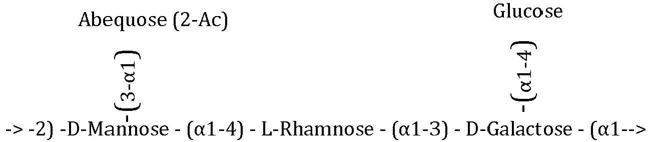

The chemical structure of O-antigens for the analogous O-antigens in other *Salmonella* species are highly similar with O-antigens from *Salmonella enterica Typhimurium* serovars.

They typically possess a main chain having a D-Man*p*-(1-->4)-L-Rha*p*-(α*1*-->3)-D-Gal*p* trisaccharide repeat unit and may differ in the configuration (α vs. β) and the position of the polymerization linkage (α 1-->2 vs. α 1-->6) and the configuration (α vs. β) of the D-Man*p*-(1-->4)-L-Rha*p* linkage (Liu et al. FEMS Microbiol Rev 38 (2014) 56-89).

The terms "nucleic acid sequence", "polynucleotide," when used in singular or plural form, generally refers to any nucleic acid sequence, polyribonucleotide or polydeoxribonucleotide, which may be unmodified RNA or DNA or modified RNA or DNA. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double- stranded or include single- and double-stranded regions. In general, the term "nucleic acid sequence" embraces all chemically, enzymatically and/or metabolically modified forms of unmodified polynucleotides, as well as the chemical forms of DNA and RNA characteristic of viruses and cells, including simple and complex cells.

The term "percent sequence identity" or "identical" in the context of nucleic acid sequences or chemical or carbohydrate sequences refers to the residues in the two sequences that are the same when aligned for maximum correspondence. The length of sequence identity comparison may be over the full- length of an open reading frame of a gene, protein, subunit, a fragment or carbohydrate.

As described herein, the percent identity among the O-antigen in proteobacteria and serovars can be about 75%, 80%, 85%, 90%, 95% or over 99%. In other embodiments, the percent identities of the O-antigen can be lower than 75%.

Identity is readily determined using such algorithms and computer programs as are defined herein at default settings. Alignments are performed using any of a variety of publicly or commercially available Multiple Sequence Alignment Programs, such as "Clustal W", accessible through Web Servers on the internet. Alternatively, Vector NTI® utilities are also used. There are also a number of algorithms known in the art that can be used to measure nucleotide sequence identity, including those contained in the programs described above. Generally, these programs are used at default settings, although one of skill in the art can alter these settings as needed. Alternatively, one of skill in the art can utilize another algorithm or computer program that provides at least the level of identity or alignment as that provided by the referenced algorithms and programs. The similarities between the sequences can also be defined as the ability to hybridize to the complement of a selected sequence, under stringent conditions.

"Isolated" means altered "by the hand of man" from its natural state, i.e., if it occurs in nature, it has been changed or removed from its original environment, or both. For example, a polynucleotide or polypeptide naturally present in a living organism is not "isolated," but the same polynucleotide or polypeptide separated from the coexisting materials of its natural state is "isolated", as the term is employed herein.

One skilled in the art may readily reproduce the compositions and methods described herein by use of the elements described herein, which are publicly available from conventional sources.

In another embodiment of the inventive immunogenic composition, said composition induces in an animal, upon infection, the release of inflammatory cytokines from macrophages or dendritic cells and an increase in the animal's innate immune response to the bacterium.

In another embodiment the immunogenic composition further comprises a pharmaceutically acceptable excipient.

In another embodiment the immunogenic composition described herein further comprises an adjuvant.

The immunogenic compositions of the present invention may be further associated with a pharmaceutically acceptable carrier for *in vivo* delivery. As used herein the term "pharmaceutically acceptable carrier" or "diluent" is intended to include any and all solvents, dispersion media, coatings, antibacterial and antifungal agents, isotonic and absorption delaying agents, and the like, compatible with administration to humans or other mammals, or animals, such as avian species. In one embodiment, the diluent is saline or buffered saline. Such pharmaceutically acceptable carriers suitable for use in such a composition are well known to those of skill in the art. Such carriers include, without limitation, and depending upon pH adjustments, buffered water, buffered saline, such as 0.8% saline, phosphate buffer, 0.3% glycine, hyaluronic acid, alcoholic/aqueous solutions, emulsions or suspensions. Other conventionally employed diluents, adjuvants and excipients, may be added in accordance with conventional techniques. Optionally, the pharmaceutical compositions can also contain a mild adjuvant, such as an aluminum salt, e.g., aluminum hydroxide or aluminum phosphate, aqueous suspensions of aluminum and magnesium hydroxides, liposomes, and oil in water emulsions.

Carriers can include ethanol, polyols, and suitable mixtures thereof, vegetable oils, and injectable organic esters. Buffers and pH adjusting agents may also be employed. Buffers include, without limitation, salts prepared from an organic acid or base. Representative buffers include, without limitation, organic acid salts, such as salts of citric acid, e.g., citrates, ascorbic acid, gluconic acid, carbonic acid, tartaric acid, succinic acid, acetic acid, or phthalic acid, Tris, trimethanmine hydrochloride, or phosphate buffers. Parenteral carriers can include sodium chloride solution, Ringer's dextrose, dextrose and sodium chloride, lactated Ringer's or fixed oils. Intravenous carriers can include fluid and nutrient replenishers, electrolyte replenishers, such as those based on Ringer's dextrose and the like. Preservatives and other additives such as, for example, antimicrobials, antioxidants, chelating agents, inert gases and the like may also be provided in the pharmaceutical carriers. These compositions are not limited by the selection of the carrier. The preparation of these pharmaceutically acceptable compositions, from the above-described components, having appropriate pH isotonicity, stability and other conventional characteristics is within the skill of the art.

The preferred features and embodiments as described above and herein and even though occasionally referred to the first aspect of the present invention apply to any and all subsequent aspects of the present invention described herein.

According to a further aspect of the present invention there is provided a method of inducing an immune response against a proteobacteria in a subject in need thereof, the method comprising administering an immunogenic composition described herein to said subject, wherein said composition is administered in an effective amount so as to elicit an immune response to said Proteobacteria. Thus, in a further aspect, the present invention provides for the inventive immunogenic composition for use in a method of inducing an immune response against a proteobacteria in a subject in need thereof, wherein said method comprises administering said inventive immunogenic composition to said subject, and wherein said composition is administered in an effective amount so as to elicit an immune response to said Proteobacteria.

According to another aspect of the present invention there is provided a method of treating a subject in need thereof against proteobacteria, the method comprising administering an immunogenic composition described herein to the subject, wherein the subject is preferably thereby resistant to one or more infections by said proteobacteria. Thus, in a further aspect, the present invention provides for the inventive immunogenic composition for use in a method of treating a subject in need thereof against proteobacteria, wherein said method comprises administering said inventive immunogenic composition to the subject, wherein the subject is preferably thereby resistant to one or more infections by said proteobacteria.

In a further aspect, the present invention provides for the inventive immunogenic composition for use as a prophylactic treatment against a disease caused by Proteobacteria in a subject, wherein preferably the subject following treatment contains only a non-transmissible form of said Proteobacteria.

According to a still further aspect of the present invention there is provided a method of lessening the severity of symptoms of proteobacteria infection in a subject in need thereof, comprising administering to the subject an effective amount of an immunogenic composition as described herein sufficient to lessen the severity of said symptoms in said subject. Thus, in a further aspect, the present invention provides for the inventive immunogenic composition for use in a method of lessening the severity of symptoms of proteobacteria infection in a subject in need thereof, wherein said method comprises administering to the subject an effective amount of said inventive immunogenic composition sufficient to lessen the severity of said symptoms in said subject.

In a further aspect the present invention provides for the inventive immunogenic composition for use in a method of inducing an immune response against a disease caused by Proteobacteria in a subject, wherein said method comprises administrating said immunogenic composition to said subject in need thereof, and wherein preferably said immune response is a protective immune response and wherein preferably administration is by intranasal, intramuscular, subcutaneous, transdermal or sublingual administration.

According to another aspect of the present invention there is provided a method of treating a subject in need thereof against proteobacteria, the method comprising administering an immunogenic composition as described herein to the subject, wherein the subject following treatment contains only a non-transmissible form of said proteobacteria. In one embodiment the subject contains only a non-transmissible form of said proteobacteria 12, 24, 48 or 72 hours following treatment. Thus, in a further aspect, the present invention provides for the inventive immunogenic composition for use in a method of lessening the severity of symptoms of proteobacteria infection in a subject in need thereof, wherein said method comprises administering to the subject an effective amount of said inventive immunogenic composition sufficient to lessen the severity of said symptoms in said subject.

According to a further aspect of the present invention there is provided a method of vaccinating a subject for a disease caused by proteobacteria, comprising administrating the immunogenic composition as described herein to a subject in need thereof by intranasal, intramuscular, subcutaneous, transdermal or sublingual administration. Thus, in a further aspect, the present invention provides for the inventive immunogenic composition for use in a method of vaccinating a subject for a disease caused by proteobacteria, wherein said method comprises administrating said inventive immunogenic composition to a subject in need thereof by intranasal, intramuscular, subcutaneous, transdermal or sublingual administration.

In another embodiment of the inventive methods, said subject is selected from a human and an agricultural animal.

In another embodiment of the inventive methods, said agricultural animal is selected from the group consisting of cattle, poultry, swine, horses, sheep and goats.

In another embodiment of the inventive methods, said agricultural animal is swine.

According to a further aspect of the present invention there is provided an immunogenic composition as described herein, for use in preventing disease caused by proteobacteria selected from the list comprising: bacterial gastroenteritis, bacterial enterocolitis, urinary tract infection, mastitis, bacterial pneumonia, bacterial sepsis. Thus, in a further aspect, the present invention provides for the inventive immunogenic composition for use in a method of preventing disease caused by proteobacteria selected from the list comprising: bacterial gastroenteritis, bacterial enterocolitis, urinary tract infection, mastitis, bacterial pneumonia, bacterial sepsis.

In a preferred embodiment, said Proteobacteria is Gammaproteobacteria. In another embodiment, the Proteobacteria may be selected from *Enterobacteriaceae, Vibrionaceae, Pseudomonadaceae, Campylobacter.* In another embodiment the Gammaproteobacteria *is Enterobacteriaceae* and said *Enterobacteriaceae* is selected from *Salmonella enteritis, Escherichia coli, Yersinia pestis, Shigella spp.*

Diseases caused by pathogenic Proteobacteria include and are typically and preferably bacterial gastroenteritis, bacterial enterocolitis, urinary tract infection, mastitis, bacterial pneumonia, bacterial sepsis.

*Salmonella* is a genus of over 2000 serovars and includes organisms that cause a wide range of human and animal diseases. For example, *Salmonella enterica* serovars Typhimurium and Enteritidis are known as the non-typhoidal *Salmonella* (NTS) and cause salmonellosis-a gastroenteritis which is usually a self-limiting illness in healthy individuals.

"Animal" or "subject" as used herein means a mammalian animal, including a human male or female, a veterinary or farm animal, e.g., horses, livestock, cattle, pigs, etc., a domestic animal or pet, e.g., dogs, cats; and animals normally used for clinical research, such as primates, rabbits, and rodents. In one embodiment, the subject is a human. In another embodiment, the subject is a swine. "Animal" as used herein is also meant to include other non- mammals or animal species that are commonly infected by *Salmonella,* such as avians or fowl that are used as food products, can be carriers for *Salmonella,* and are often the transmitters of the bacterium to humans.

According to certain methods, where the intended subject for administration is a swine, or other animal, the composition containing the inactivated and modified proteobacteria described herein may be a composition suitable to be added to a foodstuff. Depending upon the subject selected, type of animal, size, weight, general health, etc., and purpose of treatment, the mode of administration and dosage may be selected by one of skill in the art, e.g., a veterinarian or physician. For example, the mode of administration can be any suitable route: oral, subcutaneous injection, intravenous injection, intramuscular injection, mucosal, intra-arterial, intraperitoneal, parenteral, intradermal, transdermal, nasal, vaginal, or rectal or inhalation routes, among others. The term "oral" refers to administration of a compound or composition to a subject by a route or mode along the alimentary canal, such as by swallowing liquid or solid forms of a composition from the mouth. The doses may be administered as a single dose, multiple doses over a selected time gap, via prime/boosting protocols, etc.

When administered as an animal vaccine, e.g., a vaccine and immunogenic composition, respectively, for swine, for example, administration can be by gavage in a carrier. Alternatively, the bacteria can be added to gel beads and mixed with feed. Still alternatively, the bacteria can be sprayed onto the feathers or skin of the animals and inhaled. It is likely also that the bacteria can be lyophilized or otherwise treated and mixed with feed.

Similarly the selection of suitable dosages will be within the skill of the art depending upon the subject and course of treatment. Exemplary dosages may be 5 x 10⁶ - 5 x 10¹² colony forming units, e.g., 5 x 10⁵ colony forming units, or 10¹⁰-10¹¹ inactivated bacterial particles in 100 microliters for, e.g., swine. The selection of the doses and modes of administration and dosage regimens may be selected by one of skill in the art based on the subject to be vaccinated and immunized, respectively, the physical attributes of the subject, the virulence of the bacteria, the route of administration, and other common factors. Those of skill in the art will recognize that the precise dosage may vary from situation to situation and from patient to patient, depending on e.g. age, gender, overall health, various genetic factors, and other variables known to those of skill in the art. Dosages are typically determined e.g. in the course of animal and/or human clinical trials as conducted by skilled medical personnel, e.g. physicians.

As used herein, the term "effective amount" means, in the context of the inventive immunogenic composition, an amount of an immunogenic composition capable of inducing an immune response that reduces the incidence of or lessens the severity of infection or incident of disease in an animal. In a preferred embodiment the quantity, delivered by the relevant route is one in which the amount is sufficient to induce a detectable specific antibody response (as determined by bacterial flow cytometry or ELISA) in serum or mucosal secretions. Alternatively, in the context of a therapy, the term "effective amount" refers to the amount of a therapy which is sufficient to reduce or ameliorate the severity or duration of a disease or disorder, or one or more symptoms thereof, prevent the advancement of a disease or disorder, cause the regression of a disease or disorder, prevent the recurrence, development, onset, or progression of one or more symptoms associated with a disease or disorder, or enhance or improve the prophylaxis or treatment of another therapy or therapeutic agent.

The volume of a single dose of the immunogenic compositions of this invention will vary by subject and administration route but will be generally within the ranges commonly employed in conventional vaccines and immunogenic compositions, respectively. For example in juvenile swine (4-10 kg) the volume of a single oral dose is preferably between about 0.1 ml and 3 ml, preferably between about 0.2 ml and about 1.5 ml, more preferably between about 0.2 ml and about 0.5 ml at the concentrations of conjugate and adjuvant.

The preparation of compositions for use as vaccines and immunogenic compositions, respectively, is known to those of skill in the art. Typically, such compositions are prepared either as liquid solutions or suspensions, however solid forms such as tablets, pills, powders and the like are also contemplated. Solid forms suitable for solution in, or suspension in, liquids prior to administration may also be prepared (e.g. lyophilized, freeze-dried forms, etc.). The preparation may also be emulsified. The active ingredients may be mixed with excipients which are pharmaceutically acceptable and compatible with the active ingredients. Suitable excipients are, for example, water, saline, dextrose, glycerol, ethanol and the like, or combinations thereof. In addition, the composition may contain minor amounts of auxiliary substances such as wetting or emulsifying agents, pH buffering agents, and the like. If it is desired to administer an oral form of the composition, various thickeners, flavorings, diluents, emulsifiers, dispersing aids or binders and the like may be added. The composition of the present invention may contain any such additional ingredients so as to provide the composition in a form suitable for administration.

In addition, the composition may contain adjuvants, many of which are known in the art. For example, adjuvants suitable for use in the invention include but are not limited to: bacterial or microbial derivatives such as non-toxic derivatives of enterobacterial lipopolysaccharide (LPS), Lipid A derivatives, Immunostimulatory oligonucleotides e.g. containing a CpG motif (a dinucleotide sequence containing an unmethylated cytosine linked by a phosphate bond to a guanosine). Double-stranded RNAs and oligonucleotides containing palindromic or poly(dG) sequences have also been shown to be immunostimulatory.

Recipients of the inventive immunogenic compositions may have never been exposed to the specific proteobacteria for example *Salmonella,* or may have been exposed or suspected of having been exposed but be asymptomatic, or may have actual symptoms of disease, and still benefit from administration of the inventive immunogenic composition. Administration of the inventive immunogenic composition may prevent disease symptoms entirely, or may lessen or decrease disease symptoms, the latter outcome being less than ideal but still better than experiencing full-blown disease symptoms.

The invention provides methods of vaccinating, or, alternatively, of eliciting an immune response, in a subject in need thereof. The method generally involves identifying a suitable subject, and administering the immunogenic composition as described herein. The method may also encompass follow-up of administration, e.g. by assessing the production of protective antibodies by the subject, or the presence (or lack thereof) of disease symptoms, etc. The immune response that is elicited may be of any type, i.e. any type of antibody may be produced in response to administration, and cell-mediated immunity may also be elicited.

The terms "treatment"/"treating" as used herein include: (1) preventing or delaying the appearance of clinical symptoms of the state, disorder or condition developing in a subject that may be afflicted with or predisposed to the state, disorder or condition but does not yet experience or display clinical or subclinical symptoms of the state, disorder or condition; (2) inhibiting the state, disorder or condition (e.g. arresting, reducing or delaying the development of the disease, or a relapse thereof in case of maintenance treatment, of at least one clinical or subclinical symptom thereof); and/or (3) relieving the condition (i.e. causing regression of the state, disorder or condition or at least one of its clinical or subclinical symptoms). The benefit to a patient to be treated is either statistically significant or at least perceptible to the patient or to the physician. However, it will be appreciated that when a medicament is administered to a patient to treat a disease, the outcome may not always be effective treatment. In one embodiment, the terms "treatment"/"treating" as used herein, refer to a therapeutic treatment. In another embodiment, the terms "treatment"/"treating" as used herein, refer to a prophylactic treatment.

In addition, the invention provides methods of treating or preventing *Salmonella* infection by a non-typhoid *Salmonella* serovar in a subject, methods of lessening the severity of symptoms of *Salmonella* infection in a subject, and methods of decreasing fecal shedding of *Salmonella* from a subject who is or is likely to be infected with *Salmonella.* Each of these methods involves administering to the subject an effective amount of a composition the immunogenic composition described herein and a physiologically acceptable carrier. The amount of the composition is administered is sufficient to elicit an immune response to the at least one *Salmonella* serovar in said subject, thereby of treating or preventing *Salmonella* infection, lessening the severity of symptoms of *Salmonella* infection, decreasing fecal shedding of *Salmonella* and/or decreasing or preventing the transmission of the *Salmonella.*

Another aspect of the invention contemplates the inventive immunogenic composition for the protection of swine against *Salmonella* infection, wherein said immunogenic composition comprises at least two or more inactivated serovar of *Salmonella* and a pharmaceutically acceptable carrier.

Herein, suitable subjects and subjects in need to which compositions of the invention may be administered include animals in need of either prophylactic or treatment for an infection, disease, or condition.

The invention provides a method of reducing the incidence of or severity of one or more clinical signs associated with or caused by a *Salmonella* infection, comprising the step of administering an immunogenic composition of the invention as provided herewith, such that the incidence of or the severity of a clinical sign of the *Salmonella* infection is reduced by at least 10%, preferably at least 20%, even more preferred at least 30%, even more preferred at least 50%, even more preferred at least 70%, most preferred at least 100% relative to a subject that has not received the immunogenic composition as provided herein. Such clinical signs include diarrhea shedding and reduction in average daily weight gain.

As used herein, "preventative" refers to hindering or stopping a disease or condition before it occurs or while the disease or condition is still in the sub-clinical phase.

As used herein, "therapeutic" can refer to treating or curing a disease or condition.

According to another aspect of the present invention there is provided a method of generating an immunogenic composition as described herein, comprising the following steps:
i. Administer an inactivated wild type proteobacterial strain to a subject,
ii. Challenge the subject with the wildtype proteobacterial strain,
iii. Isolate clones of said proteobacterial strain from the subject between 2 hours to 7 days post infection,
iv. Identify isolated clones with reduced binding affinity to the wild type proteobacterial strain anti-O-antigen antibody,
v. Generate recombinant clones identical to said isolated clones with reduced binding affinity,
vi. Inactivate and combine the recombinant clones with the inactivated wild type proteobacterial strain.
vii. Repeat steps i-viii, administering the inactivated combined recombinant clones with the inactivated wild-type proteobacterial strain,
viii. Combine all identified inactivated clones with the inactivated wild type proteobacteria to produce the immunogenic composition.

In a preferred embodiment, the method as described in the aforementioned aspect comprises the use of bacteriophage transduction or homologous recombination to generate the recombinant clones.

### EXAMPLES

The present invention provides the following examples which are not limiting.

### Example 1

**Mice vaccinated with inactivated wild type *Salmonella enterica* serovar Typhimurium** (*S*. Typhimurium) **are weakly protected from wild-type *S*. Typhimurium challenge due to the emergence of vaccine-escape variants.**

### Generation of inactivated oral vaccines.

Peracetic acid killed vaccines were produced as previously described in Moor et al. Frontiers in Immunology (2016) Vol 7, Article 34. Briefly, bacteria were grown overnight to late stationary phase, harvested by centrifugation and resuspended to a density of 10⁹-10¹⁰ per ml in sterile PBS. Peracetic acid (Sigma-Aldrich) was added to a final concentration of 1%. The suspension was mixed thoroughly and incubated for 60min at room temperature. Bacteria were washed once in 40ml of sterile 10x PBS and subsequently three times in 50ml sterile 1x PBS. The final pellet was resuspended to yield a density of 10¹¹ particles per ml in sterile PBS (determined by OD600) and stored at 4°C for up to three weeks. As a quality control, each batch of vaccine was tested before use by inoculating 100µl of the killed vaccine (one vaccine dose) into 300ml LB and incubating over night at 37°C with aeration. Vaccine lots were released for use only when a negative enrichment culture had been confirmed.

### Vaccination and challenge of Mice

All animal experiments were approved by the legal authorities (licenses 223/2010, 222/2013 and 193/2016; Kantonales Veterinäramt Zürich, Switzerland) and performed according to the legal and ethical requirements. Vaccinations were administered between 5 and 6 weeks of age, and males and females were randomized between groups to obtain identical ratios wherever possible. Mice received either 1e10 particles of peracetic acid inactivated wild-type *S. Typhimurium* strain SL1344 ("WT vaccine", *Salmonella enterica serovar Typhimurium* strain SL1344 is available from the American Type Culture Collection) or vehicle-only control once per week per os for 4 weeks. At 28 days after the first vaccination, infections were carried out as described in Barthel, M. et al Pretreatment of mice with streptomycin provides a Salmonella enterica serovar Typhimurium colitis model that allows analysis of both pathogen and host, Infect. Immun. 71, 2839-58 (2003). In order to allow reproducible gut colonization, mice were orally pretreated 24h before infection with 25 mg streptomycin. Strains were cultivated overnight separately in LB containing the 50µg/ml streptomycin. Subcultures were prepared before infections by diluting overnight cultures 1:20 in fresh LB without antibiotic and incubation 4h at 37°C. Mice were orally challenged with live wild-type *S*. Typhimurium strain SL1344 with an inoculum size of 5^{∗}10³, 5^{∗}10⁵, and 5^{∗}10⁷.

### Analysis of the S. Typhimurium and the antibody produced in mice following challenge with wild-type S. Typhimurium strain SL1344

Fecal, mesenteric (Figure 1A) and lymph node (Figure 1B) *Salmonella* Colony Forming Units (CFU) were quantified over 2 days by plating on Mackonkey agar (Oxoid) containing relevant antibiotics.

Intestinal inflammation was quantified by measuring Lipocalin2 in feces by ELISA (R&D) in accordance with the manufacturer's instructions (Figure 1C).

*S. Typhimurium* clones were reisolated from the feces (by picking colonies from plates) of vaccinated protected and unprotected mice. These were cultivated and used as targets to titre intestinal IgA from intestinal lavages of vaccinated and unvaccinated mice by bacterial flow cytometry.

Specific antibody titers in mouse intestinal washes were measured by flow cytometry as described previously in Moor, K. et al. High-avidity IgA protects the intestine by enchaining growing bacteria. Nature 544, 498-502 (2017) and Moor, K. et al. Analysis of bacterial-surface-specific antibodies in body fluids using bacterial flow cytometry as described in Nat. Protoc. 11, 1531-1553 (2016).

### Determining antibody titres by bacterial flow cytometry

Bacterial targets (the antigens against which antibodies are to be titered) were grown to late stationary phase or the required OD, and then gently pelleted for 2 min at 3000g. The pellet was washed with sterile-filtered 1% BSA/PBS before resuspending at a density of approximately 10⁷ bacteria per ml.

To prepare mouse IgA for analysis, intestinal washes were collected by flushing the small intestine with 5ml PBS and centrifugation at 16000g for 30min. aliquots of the supernatants were stored at -20°C until analysis. For analysis, aliquots of intestinal washes were thawed, and centrifuged at 16000g for 10 min. Supernatants were used to perform serial dilutions.

25µl of the dilutions were incubated with 25µl bacterial suspension at 4°C for 1h. Bacteria were washed twice with 200µl FACS buffer before resuspending in 25µl FACS buffer containing monoclonal FITC-anti-mouse IgA (BD Pharmingen, 10µg/ml) or Brilliant violet 421-anti-IgA (BD Pharmingen). After 1h of incubation, bacteria were washed once with FACS buffer and resuspended in 300µl FACS buffer for acquisition on FACS LSRII of Beckman Coulter Cytoflex S using FSC and SSC parameters in logarithmic mode. Data were analysed using FloJo (Treestar). After gating on bacterial particles, log-median fluorescence intensities (MFI) were plotted against antibody concentrations for each sample and 4-parameter logistic curves were fitted using Prism (Graphpad, USA). Titers were calculated from these curves as the inverse of the antibody concentration giving an above-background signal.

### Results

This experiment revealed that all mice had robust IgA responses against the wild-type S. *Typhimurium* (Fig. ID). However, some mice developed intestinal inflammation and invasive disease despite a robust vaccination response, in Figure IE it was shown that the unprotected vaccinated mice had similar antibody production to vaccinated protected mice. When S. Typhimurium clones from vaccinated but unprotected mice were used to detect specific IgA in intestinal lavages, this revealed that S.Typhimurium in the intestines of these mice no longer bound vaccine-induced IgA - i.e. S. Typhimurium can escape the IgA response induced by a standard oral vaccine (Figure IF).

### Example 2

### Characterization of Salmonella variants with weak binding to standard vaccine-induced sIgA

To establish the nature of IgA escape, we first carried out Genome resequencing of *S*. Typhimurium clones from vaccinated but unprotected mice.

### Genome resequencing

The genomes of S.Tm^{wt} and evolved derivatives (*Salmonella* variants/clones) were fully sequenced by the Miseq system (2x300bp reads, Illumina, San Diego, CA) operated at the Functional Genomic Center in Zurich. The sequence of S.Tm SL1344 (NC_016810.1) was used as reference. Quality check, reads trimming, alignments, SNPs and indels calling were performed using the bioinformatics software CLC Workbench (Qiagen).

### Results

Genome sequencing revealed a common 7 base-pair contraction in the coding region of the OafA gene, known to encode an Abequose O-acetyl transferase which modifies the S. *Typhimurium* O-antigen glycan repeat (Fig. 2A). Acetylation of the O-antigen Abequose converts a serovar O4 strain into a serovar O5 strain. We therefore serotyped the reisolated clones by bacterial flow cytometry (Fig. 2B).

### Serotyping by bacterial flow cytometry

1µl of these enrichments or of primary cultures, or 1µl of fresh feces or cecal content suspension (as above) was stained with STA5 (human recombinant monoclonal IgG2 anti-012, Moor et al. Nature 2017) and Rabbit anti-Salmonella O5 (Difco). After incubation at 4°C for 30 min, bacteria were washed once with PBS/1% BSA and resuspended in appropriate secondary reagents (Alexa 647-anti-human IgG, Jackson Immunoresearch, Brilliant Violet 421-anti-Rabbit IgG, Biolegend). This was incubated for 10-60 min before cells were washed and resuspended for acquisition on a BD LSRII or Beckman Coulter Cytoflex S.

### Results

This confirmed loss of O5 on some clones (Fig. 2B).

During this flow cytometry based serotyping, a second class of clones were also identified with apparently bi-stable loss of binding to an anti-O12 typing antibody (Fig. 2C). Genome sequencing of these clones revealed no consistent mutational pattern (data not shown). We therefore carried out methylation analysis of the genome of these strains (Fig. 2D - heatmap of methylation of the promoter of the GtrABC operon).

### DNA methylation analysis

For REC-Seq (restriction enzyme cleavage-sequencing) we followed the same procedure described by Ardissone, S. et al. Cell Cycle Constraints and Environmental Control of Local DNA Hypomethylation in α-Proteobacteria. PLoS Genet. 12, e1006499 (2016). In brief, 1 µg of genomic DNA from each S.Tm was cleaved with MboI, a blocked (5'biotinylated) specific adaptor was ligated to the ends and the ligated fragments were then sheared to an average size of 150-400 bp (Fasteris SA, Geneva, CH). Illumina adaptors were then ligated to the sheared ends followed by deep-sequencing using a HiSeq Illumina sequencer, the 50 bp single end reads were quality controlled with FastQC (http://www.bioinformatics.babraham.ac.uk/projects/fastqc/). To remove contaminating sequences, the reads were split according to the MboI consensus motif (5'-^GATC-3') considered as a barcode sequence using fastx_toolkit (http://hannonlab.cshl.edu/fastx_toolkit/) (fastx_barcode_splitter.pl --bcfile barcodelist.txt -- bol --exact). A large part of the reads (60%) were rejected and 40% kept for remapping to the reference genomes with bwa mem (Li, H. & Durbin, R. Fast and accurate short read alignment with Burrows-Wheeler transform. Bioinformatics 25, 1754-1760 (2009)) and samtools (Li, H. A statistical framework for SNP calling, mutation discovery, association mapping and population genetical parameter estimation from sequencing data. Bioinformatics 27, 2987-93 (2011)) to generate a sorted bam file. The bam file was further filtered to remove low mapping quality reads (keeping AS >= 45) and split by orientation (alignmentFlag 0 or 16) with bamtools (Barnett, D. W., Garrison, E. K., Quinlan, A. R., Strömberg, M. P. & Marth, G. T. BamTools: a C++ API and toolkit for analyzing and managing BAM files. Bioinformatics 27, 1691-2 (2011)). The reads were counted at 5' positions using Bedtools (Quinlan, A. R. & Hall, I. M. BEDTools: a flexible suite of utilities for comparing genomic features. Bioinformatics 26, 841-2 (2010)) (bedtools genomecov -d -5). Both orientation count files were combined into a bed file at each identified 5'-GATC-3' motif using a homemade PERL script. The MboI positions in the bed file were associated with the closest gene using Bedtools closest (Quinlan, A. R. & Hall, I. M. BEDTools: a flexible suite of utilities for comparing genomic features. Bioinformatics 26, 841-2 (2010)) and the gff3 file of the reference genomes (Kersey, P. J. et al. Ensembl Genomes 2016: more genomes, more complexity. Nucleic Acids Res. 44, D574-D580 (2016)). The final bed file was converted to an MS Excel sheet with a homemade script. The counts were loaded in RStudio 1.1.442 (RStudio, Inc., Boston, M. RStudio: Integrated Development for R. Available at: https://www.rstudio.com/) with R version 3.4.4 (R: A Language and Environment for Statistical Computing. R Foundation for Statistical Computing. Available at: https://www.r-project.org/about.html) and analysed with the DESeq2 1.18.1 package (Love, M. I., Huber, W. & Anders, S. Moderated estimation of fold change and dispersion for RNA-seq data with DESeq2. Genome Biol. 15, 550 (2014)) comparing the reference strain with the 3 evolved strains considered as replicates. The counts are analysed by genome position rather than by gene. The positions are considered significantly differentially methylated upon an adjusted p-value < 0.05.

### Results

Of the 2607 GATC positions, only 4 were found significantly differentially methylated, and they are all located in the promoter of the gtrABC operon, which encodes a glucosyl transferase system known to add glucose to the galactose of the *S*.Typhimurium O-antigen repeat. HI NMR analysis of purified O-antigen from the reisolated clones confirmed the presence of this glucose in their O-antigen.

Therefore the *S. Typhimurium* strain we were using has two methods to escape standard vaccine-induced IgA: by mutation of a hypervariable tandem repeat it can inactivate the gene for an O-acetyl transferase, removing an acetyl group from the O-antigen repeats, and by epigenetic modification it can turn on expression of a glucosyl transferase adding a glucose to the O-antigen repeats (Broadbent, S. E., Davies, M. R. & van der Woude, M. W. Phase variation controls expression of Salmonella lipopolysaccharide modification genes by a DNA methylation-dependent mechanism. Mol. Microbiol. 77, 337-53 (2010)). Combining these two mechanisms generates four chemically different O-antigens with the serovar notations O5/O12, O4/O12, O5/O12-2 and O4/O12-2. Here we discovered the four bacterial escape variants that lead to loss of binding to antibodies induced by a wild type *Salmonella* vaccine.

### Example 3

### Strength of vaccine-mediated selective pressure on O-antigen structure.

In order to confirm that the intestinal antibodies of *Salmonella* infected mice, confer a major selective pressure for the emergence of these modifications, we carried out competitive infections.

To quantify the selective advantage of gain or loss of an acetyl group, we vaccinated mice, as described in Example 1, with vaccines constructed from O5 serovar (acetylated O-antigen) *S*.Typhimurium or from S.Typhimurium carrying an in-frame deletion of OafA (non-acetylated O-antigen, O4 serovar). On day 28, we carried out infectious challenges as described in Experiment 1, with the following modifications:
In order to specifically assay the effect of acetylation in the absence of glucosylation, we competed S.Typhimurium carrying an in-frame deletion in GtrC (acetylated O-antigen, cannot glucosylate) (O5, 012 serovar) with *S*.Typhimurium carrying in frame deletions of GtrC and OafA (non-acetylated O-antigen, cannot glucosylate) (O4, 012 serovar). The strains were mixed 1:1 prior to oral infection and carried different antibiotic resistances to all identification by differential plating. In naive or mock-vaccinated mice, the strains remain at a ratio of 1:1 in the intestinal content over 4 days of infection.

### Results

In naive mice, both serovars remained at a 1:1 ratio in intestinal content (Fig. 3A). However, mice vaccinated with the inactivated O4 serovar had an up to 10⁸-fold over-abundance of the O5 serovar over the O4 serovar (Fig. 3A). Conversely, Mice vaccinated with the inactivated O5 serovar had an up to 10⁸-fold overabundance of the O4 serovar over the O5 serovar (Fig. 3A). This selective pressure correlated strongly with the magnitude of the specific antibody response, determined by bacterial flow cytometry as in Example 1 (Fig. 3B). The assay demonstrated the key role the O-antigen-targeting IgA plays in negatively selecting *S*.Typhimurium in the gut lumen. In vaccinated mice, the presence of specific antibodies against either the acetylated O-antigen or the non-acetylated O-antigen, results in up to 1e8-fold out-competition by the non-recognised strain, indicating a very strong negative selection of the antibody-targeted strain and a huge benefit of mutation and vaccine escape.

### Testing the selective advantage of O12/O12-2 epigenetic switching

An identical experiment was carried out to assess the role of glucosylation. Mice were vaccinated with *S. Typhimurium* carrying in frame deletions of GtrC and OafA (non-acetylated O-antigen, cannot glucosylate, O4 012 serovar) or with vehicle alone. Mice were challenged with a 1:1 mixture of *S*.Typhimurium 012 serovar and 012-2 serovar (O12^{locked}O4) on an O4 background as was done in Example 1.

### Results

Mice in which the vaccine-induced IgA bound poorly to the O4/O12-2 serovar demonstrated an up to 10⁶-fold over-representation of *S*.Typhimurium able to turn on their glucosylation system, over *S*.Typhimurium genetically incapable of doing this (Fig. 3C). The assay demonstrated the key role of glucosylation of the O-antigen in IgA-mediated recognition of these bacteria. In vaccinated mice, the presence of specific antibodies against either the glucosylated O-antigen or the non-glucosylated O-antigen, results in up to 1e6-fold out-competition by the non-recognised strain (Fig. 3C), indicating a very strong negative selection of the antibody-targeted strain and a huge benefit of mutation and vaccine escape. Out competition of 012-locked strains was only observed in mice with weak 012-2 antibody response as shown in Figure 3D.

Together these experiments demonstrate the very strong selection of *S. Typhimurium* surface carbohydrates by vaccine-induced IgA.

### Example 4

### Generation of an Evolutionary Trap vaccine

By inducing IgA responses capable of recognising all of the O-antigen escape variants, we discovered that it would be possible to generate improved vaccine-mediated protection. To do this, four new strains of S.Typhimurium were constructed:
1) *S*.Typhimurium carrying an in frame deletion of GtrC (acetylated O-antigen, cannot glucosylate) (Serovar O5, O12)
2) *S*.Typhimurium carrying in frame deletions of GtrC and OafA (non-acetylated O-antigen, cannot glucosylate) (Serovar O4, O12)
3) *S*.Typhimurium carrying an in frame deletion of GtrC and a plasmid expressing the GtrABC operon under the control of a constitutive promoter (acetylated, glucosylated O-antigen) (Serovar O5, 012-2)
4) *S*.Typhimurium carrying in frame deletions of GtrC and OafA, and a plasmid carrying the GtrABC operon under the control of a constitutive promoter (non-acetylated, glucosylated O-antigen) (Serovar O4, 012-2)

Peracetic acid-inactivated vaccines were constructed from these strains and were combined at a 1:1:1:1 ratio for oral delivery, as with our standard vaccine.

At 28 days after the first vaccination, mice receiving the combined vaccine had significantly higher intestinal IgA antibody titres with specificities towards all four serovar O4/O12, O5/O12, 4/O12-2 and O5/O12-2, as determined by bacterial flow cytometry compared to unvaccinated mice (Fig. 4C).

The mice were then orally challenged as described in example 1 with wild-type S.Typhimurium. This generated robust vaccine-mediated protection, as determined by lymph node invasion of *S. Typhimurium* in naive mice versus mice that were vaccinated *(**Fig 4A**)* and intestinal inflammation in naive mice versus mice that were vaccinated (Fig. 4B). However, closer examination of the mice revealed a third evolutionary event along with acetylation and glucosylation modifications of the O-antigen; in mice receiving the combined vaccine: *S. Typhimurium* clones reisolated from the intestinal content of these mice produced only a very short variant of the O-antigen, demonstrated using bacterial flow cytometry in Figure 4D, and using polyacrylamide gel electrophoresis (using standard techniques) of purified lipopolysaccharide (Fig. 4E). Resequencing of these clones (as performed in example 2) revealed a large deletion between inverted repeats in the genome that removes the O-antigen polymerase wzyB. Thus these clones produce only a single repeat of the O-antigen. This loss of O-antigen resulted in lower binding to vaccine-induced IgA, explaining the emergence of these clones in vaccinated mice (Fig. 4F).

### The significance of selection of wzyB mutant clones

The O-antigen is normally hundreds of repeats long (i.e. contains around 400 sugar residues polymerized into a chain). This forms a thick dense hydrophilic layer around the outside of a gram negative bacterium Mostowy, R. J. & Holt, K. E. Diversity-Generating Machines: Genetics of Bacterial Sugar-Coating. Trends Microbiol. 26, 1008-1021 (2018). Chlebicz, A. & Slizewska, K. Campylobacteriosis, Salmonellosis, Yersiniosis, and Listeriosis as Zoonotic Foodborne Diseases: A Review. Int. J. Environ. Res. Public Health 15, 863 (2018). Liu, B. et al. Structural diversity in Salmonella O antigens and its genetic basis. FEMS Microbiol. Rev. 38, 56-89 (2014). When wzyB is missing, only 4 sugar residues are present in each chain making a very weak hydrophilic barrier and increasing susceptibility to complement- and pore-forming antimicrobial-mediated lysis as well as poor resistance to outer membrane stressors such as altered salt concentrations of bile acids (Murray, G. L., Attridge, S. R. & Morona, R. Altering the length of the lipopolysaccharide O antigen has an impact on the interaction of Salmonella enterica serovar Typhimurium with macrophages and complement. J. Bacteriol. 188, 2735-9 (2006). Rojas, E. R. et al. The outer membrane is an essential load-bearing element in Gram-negative bacteria. Nature 559, 617-621 (2018)).

This was demonstrated *in vitro:*

### Test 1

### Stress testing

Single or 1:1 mixture of wzyB mutant serovar with wild type S.Tm LB subcultures were diluted 1000 times in 200 µl of media distributed in 96 well Black side microplates (Costar). To measure growth and competitions in stressful conditions that specifically destabilize the outer membrane of S.Tm, a mixture of Tris and EDTA (Sigma) was diluted to final concentration (4 mM Tris, 0,4 mM EDTA) in LB. The lid-closed microplates were incubated at 37°C with fast and continuous shaking in a microplate reader (Synergy H4, BioTek Instruments). The optical density was measured at 600 nm and the green fluorescence using 491 nm excitation and 512 nm emission filter wavelengths every 10 minutes for 18 hours. The outcome of competitions was determined by calculating mean OD and fluorescence intensity measured during the last 100 minutes of incubation, this measured the survival of the bacteria following stress testing. OD and fluorescence values were corrected for the baseline value measured at time 0.

### Test 2

### Test complement resistant.

Serovar WT and Wzyb mutants were added to human serum. If the bacteria are susceptible to complement proteins of the immune system the bacteria would be lysed. Complement proteins are destroyed with heat inactivation of the human serum and this was used as a control.

Overnight LB cultures were washed three times in PBS, OD adjusted to 0,5 and incubated with human serum obtained from Unispital Basel (3 volumes of culture for 1 volume of serum) at 37°C for 1 hour. Heat inactivated (56°C, 30 min) serum was used as control treatment. Surviving bacteria were enumerated by plating on non-selective LB agar plates. For this, dilutions were prepared in PBS immediately after incubation.

### Results

WzyB-mutant clones were much more sensitive to membrane-stress-induced cell death (Fig. 5A). Additionally, Complement proteins in the serum were much more effective at killing WzyB "single repeat O-antigen" mutant S.tm than wild type S.Tm (Fig. 5B).

We also carried out in vivo competition assays with an in-frame deletion mutant for wzyB versus on a background of OAFA and GGRC deletion (short O antigen) versus a OAFA GGRC mutant producing full length antigen (long O antigen), a wild-type *S. Typhimurium* in vaccinated and naive mice, as in Example 1. We discovered that loss of full-length O-antigen is a major fitness disadvantage for *S*.Typhimurium in naive animals, but is strongly selected for in vaccinated mice (Fig. 5C). JH-/- is a mouse model that does not produce antibodies and JH +/- is the negative control mouse which can produce antibodies. The WzyB-deletion mutants are more weakly recognized by vaccine-induced antibodies due to a decrease in O-antigen present per cell (Fig. 4F), which in turn promotes propagation. Therefore our evolutionary trap vaccine forces the emergence of *S. Typhimurium* genotypes that are detrimental for transmission and for infection of unvaccinated animals.

This will be a huge benefit for disease control in high-transmission settings such as farm animal rearing, as this is the only type of vaccination against Enterobacterial pathogens that is capable of decreasing transmission efficiency.

All strains and plasmids are listed below in table 1. All primers used in the examples are listed in table 2.

**Table 1:**

| **Strains** | **Background** | **Relevant genotype** | **Source** |
|---|---|---|---|
| *S*.Tm | SL1344 14028 | Wild-Type (ATCC) Wild-Type (ATCC) | **American Type Culture Collection Hoiseth, *Nature 1981*** |
| *S.Tm^{oafA}* | SL1344 | *ΔoafA* Tag1::*aphT* | Constructed from SL1344 using primers in table 2 |
| *S*.Tm*^{gtrC}* | SL1344 | *gtrC(a)*::*cat* | Constructed from SL1344 using primers in table 2 |
| *S*.Tm*^{gtrA}* | SL1344 | *gtrA(a)::cat* | Constructed from SL1344 using primers in table 2 |
| *S*.Tm*^{oafA gtrC}* | SL1344 | *ΔoafA gtrC(a)*::*cat* | Constructed from SL1344 using primers in table 2 |
| *S*.Tm*^{oafA gtrC} kan* | SL1344 | *ΔoafA gtrC(a)*Tag1::*aphT* | Constructed from SL1344 using primers in table 2 |
| *S*.Tm*^{oafA gtrC wzyB}* | SB300 | *ΔoafA gtrC(a) wzyB::cat* | Constructed from SL1344 using primers in table 2 |
| *S*.Tm^{*oafA* ΔGT} | SB300 | *ΔoafA ΔgtrB(a) ΔgtrB(b) ΔSTM0712-0723 Δwca-wza* | Constructed from SL1344 using primers in table 2 |
| *S*.Tm*^{oafA gtrC} pGtrABC* | SL1344 | *ΔoafA gtrC(a)*::*cat* pGtrABC | Constructed from SL1344 using primers in table 2 |

| **Plasmids** | **Backbone** | **Relevant genotype** | **Reference** |
|---|---|---|---|
| pM965 | pSC101 | P*_{rpsM}-gfp* | Addgene Stecher, Infection and Immunity 2004 |
| pGtrABC | pM965 | P*_{rpsM}-gtrABC(a)* | Constructed from pM965 using primers in table 2 |
| pKD46 | Used for strain construction | | Addgene Datsenko PNAS 2000 |
| pCP20 | Used for strain construction | | Addgene Datsenko PNAS 2000 |
| pKD3 | Used for strain construction | | Addgene Datsenko PNAS 2000 |

**Table 2:**

| **Primer name** | **Sequence** | **Purpose** |
|---|---|---|
| oafA_Seq_ up | CCGCCATAGTTACGTTTTG (SEQ ID NO:3) | Sequencing of oafA |
| oafA_Seq_ dw | AAGCTATACACATAAAATAATTTGC (SEQ ID NO:4) | |
| oafA_IntSe q1 up | AGTACTTGATTTTTATATTGCAAG (SEQ ID NO:5) | |
| oafA_IntSe q2 up | GAGGTTTATGGGATAGTCC (SEQ ID NO:6) | |
| oafA_IntSe q3 up | GCCTGATATTTGCTTCCTC (SEQ ID NO:7) | |
| oafA_IntSe q4 up | CCGTAATCTGAGAGATAATGA (SEQ ID NO:8) | |
| Del_oafA_ up | AATTATAGGTAAAAAATGATCTACAAGAAATTCAGACTCGTG TGTAGGCTGGAGCTGCTTC (SEQ ID NO:9) | In frame deletion *oafA* |
| Del_oafA_ dw | GGCAAGCCCCTCTGTTTATTTTGAAATCTGCTTTTTCACTCA TATGAATATCCTCCTTAG (SEQ ID NO:10) | |
| Ver_oafA_ up | ATGTAGTTGATGTAACAGGTC (SEQ ID NO:11) | Deletion verification *oafA* |
| Ver_oafA_ dw | ATGCCCCATCAGAAAAGCT (SEQ ID NO:12) | |
| | | |
| Ver_STM0 558_up | ATTGGTGTGATAAATCCTATTG (SEQ ID NO:13) | Deletion verification *gtrC*(a) |
| Ver_STM0 558_dw | GCTATCAGCCTGATATGCG (SEQ ID NO:14) | |
| Ver_STM4 205_up | GTAATCATCAGAGTGAATAGG (SEQ ID NO:15) | Deletion verification *gtrC*(b) |
| Ver_STM4 205_ dw | CGCAATTAGCCTTATTTGCG (SEQ ID NO:16 | |
| Del_wca_w za_up | TAAAAATAGCGGTACTTACCCTCCCCGCTTCGGCAGCGAAT GTGTAGGCTGGAGCTGCTTC (SEQ ID NO:17) | Deletion cluster *wca-wza* |
| Del_wca_w za dw | AGTGATAAATAATCAATGATGAAATCCAAAATGAAATTGACA TATGAATATCCTCCTTAG (SEQ ID NO:18) | |
| Ver_wca_ wza_up | CCATAACATTAAGTATGAACAACT (SEQ ID NO:19) | Verification deletion cluster *wca-wza* |
| Ver_wca_ wza dw | AAGCCGCTATTTAAATTGCACA (SEQ ID NO:20) | |
| Del_0712-23_up | TGATGGATTTGTTTTGTGAAAAGAAAATATCTTACGCAAGTG TGTAGGCTGGAGCTGCTTC (SEQ ID NO:21) | Deletion cluster SaltsV1_0712 to SaltsV1_0723 |
| Del_0712-23_ dw | GGAATTAAATGACGCTTAGTTATATTTTGCCCAAAATTTTCA TATGAATATCCTCCTTAG (SEQ ID NO:22) | |
| Ver_0712-23_up | ATTAAACTCATCTGATCAGTGAT (SEQ ID NO:23) | Verification deletion cluster SaitsV1_0712 to SaltsV1_0723 |
| Ver_0712-23_ dw | GGCGAGCGCCCAATAAT (SEQ ID NO:24) | |
| | | |
| Del_0559_ up | CGACTAACGAGATTTTCATTTCGCATCCCTAAAGACAATGT GTGTAGGCTGGAGCTGCTTC (SEQ ID NO:25) | In frame deletion *gtrA(a)* |
| Del_0559_ dw | CCGCTGATTTTCATAATGTTGAAGTTATTCGCTAAGTACACA TATGAATATCCTCCTTAG (SEQ ID NO:26) | |
| Ver_0559_up | TAGAAAATAGGTATCGTGGCT (SEQ ID NO:27) | Verification deletion *gtrA(a)* |
| Ver_0559_ dw | GTAGTGCTACACTCCAGAC (SEQ ID NO:28) | |
| Del_gtrC_u p | ATAATTAAGAATGAGAAGAAAAATGGTTAACAATAGATTATG TGTAGGCTGGAGCTGCTTC (SEQ ID NO:29) | In frame deletion *gtrC(a)* |
| Del_gtrC_d w | TACATGAATGTTATTTAATTATTTCCGTAATATTCTCATTCAT ATGAATATCCTCCTTAG (SEQ ID NO:30) | |
| Ver_gtrC_u p | CGCCCGTTACCCATTGG (SEQ ID NO:31) | Verification deletion *gtrC(a)* |
| Ver_gtrC_d w | TTGATAGGAATAGGTATTCTTGG (SEQ ID NO:32) | |
| | | |
| Del_wzyB_ up | TTCTAAAGGCTCTATATGCTTATAATTTCATACATTGCATTAT GCTGTGCAGGCTGGAGCTGCTTC (SEQ ID NO:33) | In frame deletion *wzyB* |
| Del_wzyB_ dw | TTGCCGCCGTATAACTTATTTATTGTTTCTTAGTAAAACGAA TCTCATATGAATATCCTCCTTAG (SEQ ID NO:34) | |
| Ver_wzyB_ up | CCAACAAGCTTTACAGGAAC (SEQ ID NO:35) | Verification deletion *wzyB* |
| Ver_wzyB_ dw | GATTCAGAATATCTTGCCAGA (SEQ ID NO:36) | |
| | | |
| PstI_Gtr57. 59_up | ATCGTACTGCAGATGTTGAAGTTATTCGCTAAGTA (SEQ ID NO:37) | Cloning *gtrABC(a)* |
| EcoRV_Gtr 57.59_ dw | GTAATCGATATCGGCGGGGAACATTAATTATAC (SEQ ID NO:38) | |
| SeqInt1_gt rABC | CATACATCCTCTATTACTCATC (SEQ ID NO:39) | Sequencing *PrpsM-gtrABC(a)* |
| SeqInt2_gt rABC | ATCTCTTGTAGTTGTATTAATTTCT (SEQ ID NO:40) | |
| SeqInt3_gt rABC | TAATTAAGAATGAGAAGAAAAATGGT (SEQ ID NO:41) | |
| SeqInt4_gt rABC | GGTGCTGGCTAAGCGC (SEQ ID NO:42) | |
| SeqInt5_gt rABC | CAGCTGTCTTACGCTTCAT (SEQ ID NO:43) | |
| SeqInt6_gt rABC | ATCAGCCTGATATGCGGATT (SEQ ID NO:44) | |

## Claims

1. An immunogenic composition comprising at least two or more inactivated serovar of a Proteobacteria strain, wherein each of said two or more inactivated serovar comprises a genetic modification of the O-antigen, wherein independently each of said genetic modification comprises a glucosylation and/or O-acetylation of the O-antigen.

2. The immunogenic composition according to claim 1, wherein the composition additionally comprises the inactivated wild type serovar of said Proteobacteria strain.

3. An immunogenic composition according to claims 1 or 2, wherein the genetic modification of the O-antigen are created following deletions and/or epigenetic modifications of the O-antigen modifying genes Abequose O-acetyl transferase (OafA gene) and gtrABC operons (gtrC gene).

4. The immunogenic composition according to claims 1 to 3, wherein the immunogenic composition induces in an animal, upon infection with the wild type serovar of said Proteobacteria, the production of a serovar of said Proteobacteria strain with a decreased virulence, compared to animals not exposed to the immunogenic composition, wherein decreased virulence is measured as a decrease in mortality and/or morbidity of the infected animal.

5. The immunogenic composition according to any one of claims 1-4, wherein the immunogenic composition induces in an animal, upon infection with the wild type serovar of said Proteobacteria, the production of a serovar of said Proteobacteria strain with a decreased transmission rate, compared to animals not exposed to the immunogenic composition.

6. The immunogenic composition according to anyone of claims 1-5, wherein the Proteobacteria is *Salmonella spp* or *Escherichia coli,* preferably wherein the Proteobacteria is *Salmonella enterica.*

7. The immunogenic composition according to claim 6, wherein the *Salmonella enterica is Salmonella enterica subspecies enterica* serovar Typhimurium.

8. The immunogenic composition according to claim 7, wherein the *Salmonella enterica subspecies enterica Typhimurium is serovar Typhimurium* strain ATCC SL1344 or ATCC 14028.

9. The immunogenic composition according to any one of claims 1-8, wherein the Proteobacteria is *Salmonella enterica serovar Typhimurium,* and wherein said at least 2 inactivated serovar of said Proteobacteria strain are selected from O5 012 serovar, O4 012 serovar, O5 012-2 serovar and O4, 012-2 serovar.

10. The immunogenic composition according to any one of claims 1-9, wherein the Proteobacteria is *Salmonella enterica subspecies enterica serovar Typhimurium,* and wherein said composition comprises at least 4, preferably exactly 4, inactivated serovar of said Proteobacteria strain, and wherein said 4 inactivated serovar are O5 012 serovar, O4 012 serovar, O5 012-2 serovar and O4, 012-2 serovar, and wherein preferably the O-antigen of said four inactivated serovar comprises a glycan structure selected from the following formula:
O5 O12 serovar: 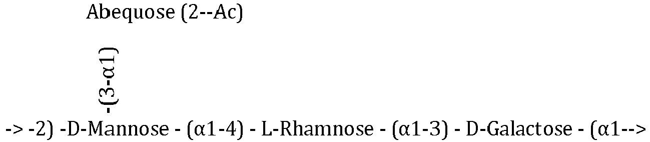
O4 O12 serovar: 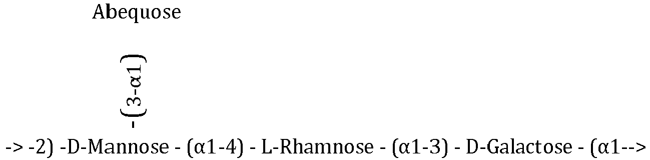
O5 012-2 serovar: 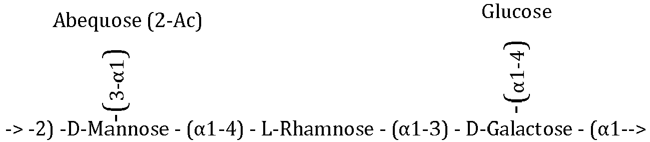 and
O4, 012-2 serovar:

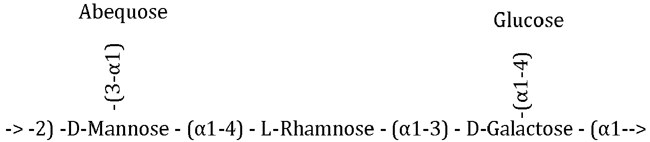

11. The immunogenic composition according to anyone of claims 1-10 for use as a prophylactic treatment against a disease caused by Proteobacteria in a subject, wherein preferably the subject following treatment contains only a non-transmissible form of said Proteobacteria.

12. The immunogenic composition according to anyone of claims 1-10 for use in a method of inducing an immune response against a disease caused by Proteobacteria in a subject, wherein said method comprises administrating said immunogenic composition to said subject in need thereof, and wherein preferably administration is by intranasal, intramuscular, subcutaneous, transdermal or sublingual administration.

13. The immunogenic composition for use according to claims 11 or 12, wherein said subject is selected from a human and an agricultural animal, preferably wherein said agricultural animal is selected from the group consisting of: cattle, poultry, swine, horses, sheep and goats.

14. An immunogenic composition according to anyone of claims 1-10, for use in preventing a disease caused by Proteobacteria selected from bacterial gastroenteritis, bacterial enterocolitis, urinary tract infection, mastitis, bacterial pneumonia and bacterial sepsis.

15. A method of generating an immunogenic composition according to any one of claims 1-10, comprising the following steps:
i. Administer an inactivated wild type Proteobacterial strain to a subject,
ii. Challenge the subject with the wildtype Proteobacterial strain,
iii. Isolate clones of said Proteobacterial strain from the subject between 2 hours to 7 days post infection,
iv. Identify isolated clones with reduced binding affinity to the wild type Proteobacteria anti-O-antigen antibody,
v. Generate recombinant clones identical to said isolated clones with reduced binding affinity,
vi. Inactivate and combine the recombinant clones with the inactivated wild type Proteobacterial strain,
vii. Repeat steps i-viii, administering the inactivated combined recombinant clones with the inactivated wild-type Proteobacterial strain,
viii. Combine all identified inactivated clones with the inactivated wild type Proteobacteria to produce the immunogenic composition.


SEQUENCE LISTING

<110>  ETH Zurich

<120>  NEW IMMUNOGENIC COMPOSITIONS

<130>  P5726EP

<160>  46

<170>  PatentIn version 3.5

<210>  1
<211>  75
<212>  DNA
<213>  Salmonella typhimurium

<400>  1
gtcactatca gttgagtggc aattttatat tttatatcct ttattagtca ttatagttaa      60

aaaattacgg tttcc                                                        75

<210>  2
<211>  68
<212>  DNA
<213>  Salmonella typhimurium

<400>  2
   gtcactatca gttgagtggc aattttatat cctttattag tcattatagt taaaaaatta      60
   cggtttcc                                                                68

<210>  3
<211>  19
<212>  DNA
<213>  Salmonella typhimurium

<400>  3
ccgccatagt tacgttttg                                                    19

<210>  4
<211>  25
<212>  DNA
<213>  Salmonella typhimurium

<400>  4
aagctataca cataaaataa tttgc                                             25

<210>  5
<211>  24
<212>  DNA
<213>  Salmonella typhimurium

<400>  5
   agtacttgat ttttatattg caag                                               24

<210>  6
<211>  19

<212>  DNA
<213>  Salmonella typhimurium

<400>  6
gaggtttatg ggatagtcc                                                    19


<210>  7
<211>  19
<212>  DNA
<213>  Salmonella typhimurium

<400>  7
gcctgatatt tgcttcctc                                                    19


<210>  8
<211>  21
<212>  DNA
<213>  Salmonella typhimurium

<400>  8
ccgtaatctg agagataatg a                                                 21


<210>  9
<211>  61
<212>  DNA
<213>  Salmonella typhimurium

<400>  9
aattataggt aaaaaatgat ctacaagaaa ttcagactcg tgtgtaggct ggagctgctt      60

c                                                                       61


<210>  10
<211>  60
<212>  DNA
<213>  Salmonella typhimurium

<400>  10
ggcaagcccc tctgtttatt ttgaaatctg ctttttcact catatgaata tcctccttag      60


<210>  11
<211>  21
<212>  DNA
<213>  Salmonella typhimurium

<400>  11
atgtagttga tgtaacaggt c                                                 21


<210>  12
<211>  19
<212>  DNA
<213>  Salmonella typhimurium

<400>  12
atgccccatc agaaaagct                                                    19

<210>  13
<211>  22
<212>  DNA
<213>  Salmonella typhimurium

<400>  13
attggtgtga taaatcctat tg                                                22

<210>  14
<211>  19
<212>  DNA
<213>  Salmonella typhimurium

<400>  14
gctatcagcc tgatatgcg                                                    19

<210>  15
<211>  21
<212>  DNA
<213>  Salmonella typhimurium

<400>  15
gtaatcatca gagtgaatag g                                                 21

<210>  16
<211>  20
<212>  DNA
<213>  Salmonella typhimurium

<400>  16
cgcaattagc cttatttgcg                                                   20

<210>  17
<211>  61
<212>  DNA
<213>  Salmonella typhimurium

<400>  17
taaaaatagc ggtacttacc ctccccgctt cggcagcgaa tgtgtaggct ggagctgctt        60

c                                                                       61

<210>  18
<211>  60
<212>  DNA
<213>  Salmonella typhimurium

<400>  18
agtgataaat aatcaatgat gaaatccaaa atgaaattga catatgaata tcctccttag        60

<210>  19
<211>  24
<212>  DNA
<213>  Salmonella typhimurium

<400>  19
ccataacatt aagtatgaac aact                                              24

<210>  20
<211>  22
<212>  DNA
<213>  Salmonella typhimurium

<400>  20
aagccgctat ttaaattgca ca                                                22


<210>  21
<211>  61
<212>  DNA
<213>  Salmonella typhimurium

<400>  21
tgatggattt gttttgtgaa aagaaaatat cttacgcaag tgtgtaggct ggagctgctt      60

c                                                                       61


<210>  22
<211>  60
<212>  DNA
<213>  Salmonella typhimurium

<400>  22
ggaattaaat gacgcttagt tatattttgc ccaaaatttt catatgaata tcctccttag      60


<210>  23
<211>  23
<212>  DNA
<213>  Salmonella typhimurium

<400>  23
attaaactca tctgatcagt gat                                               23


<210>  24
<211>  17
<212>  DNA
<213>  Salmonella typhimurium

<400>  24
ggcgagcgcc caataat                                                      17


<210>  25
<211>  61
<212>  DNA
<213>  Salmonella typhimurium

<400>  25
cgactaacga gattttcatt tcgcatccct aaagacaatg tgtgtaggct ggagctgctt      60

c                                                                       61


<210>  26
<211>  60
<212>  DNA

<213>  Salmonella typhimurium

<400>  26
ccgctgattt tcataatgtt gaagttattc gctaagtaca catatgaata tcctccttag      60


<210>  27
<211>  21
<212>  DNA
<213>  Salmonella typhimurium

<400>  27
tagaaaatag gtatcgtggc t                                                21


<210>  28
<211>  19
<212>  DNA
<213>  Salmonella typhimurium

<400>  28
gtagtgctac actccagac                                                   19


<210>  29
<211>  61
<212>  DNA
<213>  Salmonella typhimurium

<400>  29
ataattaaga atgagaagaa aaatggttaa caatagatta tgtgtaggct ggagctgctt      60

c                                                                      61


<210>  30
<211>  60
<212>  DNA
<213>  Salmonella typhimurium

<400>  30
tacatgaatg ttatttaatt atttccgtaa tattctcatt catatgaata tcctccttag      60


<210>  31
<211>  17
<212>  DNA
<213>  Salmonella typhimurium

<400>  31
cgcccgttac ccattgg                                                     17


<210>  32
<211>  23
<212>  DNA
<213>  Salmonella typhimurium

<400>  32
ttgataggaa taggtattct tgg                                              23


<210>  33

<211>  66
<212>  DNA
<213>  Salmonella typhimurium

<400>  33
ttctaaaggc tctatatgct tataatttca tacattgcat tatgctgtgc aggctggagc      60

tgcttc                                                                  66


<210>  34
<211>  65
<212>  DNA
<213>  Salmonella typhimurium

<400>  34
ttgccgccgt ataacttatt tattgtttct tagtaaaacg aatctcatat gaatatcctc      60

cttag                                                                   65


<210>  35
<211>  20
<212>  DNA
<213>  Salmonella typhimurium

<400>  35
ccaacaagct ttacaggaac                                                   20


<210>  36
<211>  21
<212>  DNA
<213>  Salmonella typhimurium

<400>  36
gattcagaat atcttgccag a                                                 21


<210>  37
<211>  35
<212>  DNA
<213>  Salmonella typhimurium

<400>  37
atcgtactgc agatgttgaa gttattcgct aagta                                  35


<210>  38
<211>  33
<212>  DNA
<213>  Salmonella typhimurium

<400>  38
gtaatcgata tcggcgggga acattaatta tac                                    33


<210>  39
<211>  22
<212>  DNA
<213>  Salmonella typhimurium

<400>  39

catacatcct ctattactca tc                                                22

<210>  40
<211>  25
<212>  DNA
<213>  Salmonella typhimurium

<400>  40
atctcttgta gttgtattaa tttct                                             25

<210>  41
<211>  26
<212>  DNA
<213>  Salmonella typhimurium

<400>  41
taattaagaa tgagaagaaa aatggt                                            26

<210>  42
<211>  16
<212>  DNA
<213>  Salmonella typhimurium

<400>  42
ggtgctggct aagcgc                                                       16

<210>  43
<211>  19
<212>  DNA
<213>  Salmonella typhimurium

<400>  43
cagctgtctt acgcttcat                                                    19

<210>  44
<211>  20
<212>  DNA
<213>  Salmonella typhimurium

<400>  44
atcagcctga tatgcggatt                                                   20

<210>  45
<211>  25
<212>  PRT
<213>  Salmonella typhimurium

<400>  45

Ser Leu Ser Val Glu Trp Gln Phe Tyr Ile Leu Tyr Pro Leu Leu Val
1               5                   10                  15

Ile Ile Val Lys Lys Leu Arg Phe Pro
            20                  25

<210>  46
<211>  15
<212>  PRT
<213>  Salmonella typhimurium

<400>  46

Gly His Tyr Gln Leu Ser Gly Asn Phe Ile Phe Tyr Ile Leu Tyr
1               5                   10                  15